# EUROPÄISCHE PATENTSCHRIFT

(11) **EP 3 126 489 B1**
(45) Veröffentlichungstag und Bekanntmachung des Hinweises auf die Patenterteilung: **12.09.2018**
(21) Anmeldenummer: 15716726.3
(22) Anmeldetag: 27.03.2015
(51) Int. Cl.: C12N 5/00

(54) **PHYSIKALISCHES SELBSTORGANISIERENDES HYDROGELSYSTEM FÜR BIOTECHNOLOGISCHE ANWENDUNGEN**
PHYSICAL SELF-ORGANIZING HYDROGEL SYSTEM FOR BIOTECHNOLOGICAL APPLICATIONS
SYSTÈME D'HYDROGEL PHYSIQUE AUTO-ORGANISATEUR POUR DES APPLICATIONS BIOTECHNOLOGIQUES

(30) Priorität: 01.04.2014 DE 102014104566
(43) Veröffentlichungstag der Anmeldung: 08.02.2017
(73) Patentinhaber: Leibniz-Institut für Polymerforschung Dresden e.V., 01069 Dresden (DE); Technische Universität Dresden, 01069 Dresden (DE)
(72) Erfinder: THOMPSON, Michael, 01099 Dresden (DE); WIEDUWILD, Robert, 01069 Dresden (DE); REDDAVIDE, Francesco, 01099 Dresden (DE); TSURKAN, Mikhail, 01217 Dresden (DE); ANDRADE, Helena, 01309 Dresden (DE); WERNER, Carsten, 01324 Dresden (DE); ZHANG, Yixin, 01219 Dresden (DE)
(74) Vertreter: Sperling, Thomas
(86) Internationale Anmeldenummer: PCT/DE2015/100130
(87) Internationale Veröffentlichungsnummer: WO 2015/149753

(56) Entgegenhaltungen:
- EP-A1- 2 594 292
- WO-A1-2012/155110
- HUAPING TAN ET AL: "Injectable Nanohybrid Scaffold for Biopharmaceuticals Delivery and Soft Tissue Engineering", MACROMOLECULAR RAPID COMMUNICATIONS, Bd. 33, Nr. 23, 13. Dezember 2012 (2012-12-13), Seiten 2015-2022, XP055200555, ISSN: 1022-1336, DOI: 10.1002/marc.201200360
- LIU Y ET AL: "Biodegradable PEG hydrogels cross-linked using biotin-avidin interactions", AUSTRALIAN JOURNAL OF CHEMISTRY 2010 CSIRO AUS, Bd. 63, Nr. 10, 2010, Seiten 1413-1417, XP008176911, DOI: 10.1071/CH10168
- RYAN G. WYLIE ET AL: "Spatially controlled simultaneous patterning of multiple growth factors in three-dimensional hydrogels", NATURE MATERIALS, Bd. 10, Nr. 10, 28. August 2011 (2011-08-28), Seiten 799-806, XP055200315, ISSN: 1476-1122, DOI: 10.1038/nmat3101
- CLAIRE E. CHIVERS ET AL: "How the biotin-streptavidin interaction was made even stronger: investigation via crystallography and a chimaeric tetramer", BIOCHEMICAL JOURNAL, Bd. 9, Nr. 1, 15. März 2011 (2011-03-15), Seiten 582-63, XP055200533, ISSN: 0264-6021, DOI: 10.1021/ac9008447
- CLAIRE E CHIVERS ET AL: "A streptavidin variant with slower biotin dissociation and increased mechanostability", NATURE METHODS, NATURE PUBLISHING GROUP, GB, Bd. 7, Nr. 5, 1. Mai 2010 (2010-05-01), Seiten 391-393, XP002617243, ISSN: 1548-7091, DOI: 10.1038/NMETH.1450 [gefunden am 2010-04-11]
- MICHAEL S. THOMPSON ET AL: "Self-Assembling Hydrogels Crosslinked Solely by Receptor-Ligand Interactions: Tunability, Rationalization of Physical Properties, and 3D Cell Culture", CHEMISTRY - A EUROPEAN JOURNAL, Bd. 21, Nr. 8, 16. Februar 2015 (2015-02-16), Seiten 3178-3182, XP055200210, ISSN: 0947-6539, DOI: 10.1002/chem.201406366
- BORSTING C ET AL: "SNP TYPING ON THE NANOCHIP ELECTRONIC MICROARRAY", METHODS IN MOLECULAR BIOLOGY, HUMANA PRESS, INC, US, Bd. 297, 1. November 2004 (2004-11-01), Seiten 155-167, XP001248547, ISSN: 1064-3745
- HIROYUKI INOUE ET AL: "Disintegration of Layer-by-Layer Assemblies Composed of 2-Iminobiotin-Labeled Poly(ethyleneimine) and Avidin", BIOMACROMOLECULES, Bd. 6, Nr. 1, 1. Januar 2005 (2005-01-01), Seiten 27-29, XP055211842, ISSN: 1525-7797, DOI: 10.1021/bm0495856

## Beschreibung

Die Erfindung betrifft ein physikalisches selbstorganisierendes Hydrogelsystem für biotechnologische Anwendungen. Die Hydrogelbildung erfolgt dabei mit kontrollierter Kinetik auf der Grundlage einer Protein-Ligand-Wechselwirkung.

Die Avidin/Streptavidin-Biotin-Interaktion ist das am meisten verwendete Protein-Liganden-Paar in der Biotechnologie. Biomaterialien auf Basis der Avidin/Streptavidin-Biotin-Interaktion finden zum Beispiel Anwendung in der Zellkultur oder zur kontrollierten Wirkstofffreisetzung. In der Forschung werden bislang chemische Reaktionen genutzt, um Biotin oder Streptavidin in ein Material oder auf einer Oberfläche zu inkorporieren. Vor kurzer Zeit wurde ein kovalentes Hydrogel-System entwickelt, das mit einer 2-Photonenphotochemischen Reaktion kombiniert ist. Diese Methode ermöglicht den Einschluss von biotinylierten Substanzen in 3D-Matrizen. Für viele zellbasierte Anwendungen sind Methoden, die auf chemische Reaktionen verzichten, von Vorteil, um Biofunktionen zu integrieren.

Chemische Reaktionen sind jedoch über einen weiten Bereich nur schwer kontrollierbar und wenig ergiebig. Es ist allerdings auch sehr schwierig, ein physikalisches Hydrogel zu bilden, das beispielsweise auf einer Avidin/Streptavidin-Biotin-Interaktion basiert, da diese Komponenten in Verbindung oft zur Bildung von Präzipitaten und Aggregaten neigen.

Aus der Druckschrift HUAPING TAN ET AL: "Injectable Nanohybrid Scaffold for Biopharmaceuticals Delivery and Soft Tissue Engineering", MACROMOLECULAR RAPID COMMUNICATIONS, Bd. 33, Nr. 23, 13. Dezember 2012 (2012-12-13), Seiten 2015-2022, XP055200555, ISSN: 1022-1336, DOI: 10.1002/marc.201200360**;** sind Nanofiber-Hydrogel-Trägermaterialien zur Einkapselung von Zellen und zum Einsatz in der Gewebetechnologie bekannt. Diese werden durch Konjugation von Biotinenden, die an PEG-Moleküle gebunden sind, an Streptavidingruppen von mit Streptavidin funktionalisierter Hyaluronsäure hergestellt.

In der Druckschrift LIU Y ET AL: "Biodecradable PEG hydrogels cross-linked using biotin-avidin interactions", AUSTRALIAN JOURNAL OF CHEMISTRY 2010 CSIRO AUS, Bd. 63, Nr. 10, 2010, Seiten 1413-1417, XP008176911, DOI: 10.1071/CH10168**;** sind biologisch abbaubare PEG-Hydrogele beschrieben, die durch Biotin-Avidin-Wechselwirkungen miteinander vernetzt sind.

In der Druckschrift BORSTING C. ET AL: "SNP TYPING ON THE NANOCHIP ELECTRONIC MICROARRAY", METHODS IN MOLECULAR BIOLOGY, HUMANA PRESS, INC, US, Bd. 297, 1. November 2004 (2004-11-01), Seiten 155-167, XP001248547, ISSN: 1064-3745**;** ist ein Einzelnukleotid-Polymorphismus-Typisierungsprotokoll (SNP) offenbart, welches für ein elektronisches Nanochip-Mikroarray entwickelt wurde. Dabei besteht das Nanochip-Array aus hundert Elektroden, die durch eine dünne Hydrogelschicht abgedeckt sind, welche Streptavidin enthält. Eine elektrische Stromstärke kann an einer, mehreren oder allen Elektroden zur gleichen Zeit angelegt werden. Biotinylierte DNA wird über die elektronischen Felder ausgerichtet und an das Streptavidin in der Hydrogelschicht gebunden.

Aus der WO 2012/155110 A1 sind Mikrogele und Mikrogewebe für den Einsatz in der Gewebetechnologie bekannt. Insbesondere ist eine Mikrokapselungsvorrichtung zur Herstellung von Mikrogelen und/oder Mikrogeweben mittels einer Emulsionstechnologie beschrieben. Außerdem werden Methoden zur Herstellung höhergeordneter Strukturen vorgestellt, die In-vivo-Gewebestrukturen nachahmen.

Aus der Druckschrift HIROYUKI INOUE ET AL: "Disintegration of Layer-by-Layer Assemblies Composed of 2-Iminobiotin-Labeled Poly(ethyleneimine) and Avidin", BIOMACROMOLECULES, Bd. 6, Nr. 1, 1. Januar 2005 (2005-01-01), Seiten 27-29, XP055211842, ISSN: 1525-7797, DOI: 10.1021/bm0495856**;** ist ein schichtartig aufgebauter Dünnfilm beschrieben, der aus Avidin und einem 2-Iminobiotin-markierten Polyethylenimin (ib-PEI) hergestellt wird und dessen Empfindlichkeit gegenüber dem Umgebungs-pH-Wert und Biotin untersucht wird.

Aufgabe der Erfindung ist es, solche Bedingungen zu schaffen, die die Erzeugung von kleinen Polymerpartikeln verhindern, sowie die Bildung eines kohärenten Hydrogels oder eines Biofilms ermöglichen.

Die Lösung der Aufgabe der Erfindung besteht in einem physikalischen selbstorganisierenden Hydrogelsystem für biotechnologische Anwendungen nach Anspruch 1. Dieses besteht aus einem nicht-kovalenten Netzwerk auf der Grundlage einer Protein-Ligand-Wechselwirkung und umfasst Biotin oder eines seiner Derivate als Ligand, wobei Biotin oder eines seiner Derivate kovalent jeweils an ein Ende einer Polymerkette eines Einzel- oder Doppelstrang-Oligonukleotids konjugiert ist, und ein tetrameres Protein als Protein, wobei der Feststoffgehalt bezogen auf das gesamte Hydrogel mindestens 3, vorzugsweise mindestens 4 Massen-%, beträgt und die Konjugate durch das tetramere Protein vernetzt sind. Erfindungsgemäß ist ein Mischungsverhältnis als Äquivalentverhältnis zwischen dem Protein und der Anzahl der terminalen biotinylierten Enden der Polymerketten im Biotin-Polymer-Konjugat von 1:2 bis 1:8 vorgesehen. Bevorzugt ist ein Äquivalentverhältnis von 1:4, was bei zwei terminalen Enden pro Polymerkette einem molaren Verhältnis von 1:2 zwischen Protein und Polymer-Biotin-Konjugat entspricht. Als die Konjugate vernetzendes tetrameres Protein ist vorzugsweise Avidin, Streptavidin oder eine Mutante dieser Proteine vorgesehen. Besonders vorteilhaft ist ein Feststoffgehalt von mindestens 6 Massen-%, vorzugsweise mindestens 9 Massen-%. Als die Konjugate vernetzendes tetrameres Protein ist vorzugsweise Avidin, Streptavidin oder eine Mutante dieser Proteine vorgesehen. Es sollten daher vorzugsweise nicht mehrere dieser Proteine miteinander kovalent verbunden sein.

Die Netzwerkbildung wird durch die Prozesse der physikalisch-chemischen Wechselwirkungen zwischen den Polymerketten gesteuert.

Überraschenderweise führen das gewählte Moleküldesign und die erfindungsgemäßen Vernetzungsbedingungen dazu, dass die Erzeugung von kleinen Polymerpartikeln verhindert wird und es zur Bildung eines zusammenhängenden Hydrogels oder eines Bio-Matrixfilms kommt.

Mit der vorteilhaften Verwendung von Traptavidin, einer mutierten Form von Streptavidin, kann die Hydrogel-Stabilität darüber hinaus im Vergleich zu Streptavidin/Avidin-Polymer-Biotin-Konjugat-Systemen bemerkenswert erhöht werden.

Gemäß der Erfindung ist ein Oligonukleotid als Polymerkette vorgesehen, das gemäß einer vorteilhaften Ausgestaltung als ein verzweigtes Oligonukleotid, das heißt ein Oligonukleotid mit mehr als zwei Enden, eingesetzt werden kann. In einer besonders bevorzugten Ausführungsform der Erfindung besteht die Oligonukleotid-Polymerkette aus einer doppelsträngigen DNA, das heißt, an Stelle eines Einzelstrang-Oligonukleotids wird doppelsträngige DNA eingesetzt. Dies ermöglicht einen noch kontrollierteren Gelierungsprozess.

Die optimierten Methoden verbessern nicht nur die Hydrogelstabilität, sondern ermöglichen auch die Bildung dünner Hydrogelfilme bei minimalem Substanzeinsatz. Das System kann somit verwendet werden, um dünne Hydrogel-Filme zu bilden, deren Herstellung kostengünstig ist, was die Beschichtung großer Oberflächen mit den gewünschten Biomaterialien ermöglicht. Das Hydrogelsystem bildet ebenso eine einfache Plattform, um viele Biomatrix-Filme mit unterschiedlichen Bedingungen zu erzeugen und damit zum Beispiel ein Hochdurchsatz-Screening durchführen zu können. Ein weiteres interessantes Merkmal des neuen Hydrogel-Systems ist dessen Fähigkeit, nach dem Trocknen wieder vollständig hergestellt werden zu können. Diese Eigenschaft ermöglicht einen leichten Materialtransport.

Das erfindungsgemäße Hydrogelsystem kann *in situ* ohne chemische Reaktionen gebildet werden. Das ist neu im Vergleich zu bereits bekannten Hydrogelen auf der Basis von Streptavidin-Biotin-Interaktionen. Die Erosion kann durch Zugabe von Biotin ohne Proteasen beschleunigt werden. Eine mögliche Anwendung besteht in der Analyse intakter extrazellulärer Matrixproteine, die von im Hydrogel eingebetteten Zellen segretiert werden.

Ein weiterer Aspekt der Erfindung betrifft ein Netzbildungsverfahren, das auf einer Biotin-Avidin-Wechselwirkung basiert. Die Netzwerkbildung wird durch die Prozesse der physikalisch-chemischen Wechselwirkungen zwischen den Polymerketten gesteuert. Wie bereits erwähnt, konnte ein Moleküldesign und Vernetzungsbedingungen ermittelt werden, die zur Verhinderung der Erzeugung von kleinen Polymerpartikeln führen und die Bildung eines zusammenhängenden Hydrogels oder eines Bio-Matrixfilms ermöglichen. Bei dem Verfahren zur Herstellung des erfindungsgemäßen Hydrogels, einschließlich aller seiner genannten Ausführungsformen, werden die zu vernetzenden Komponenten, das heißt das Protein und das Konjugat, bei einem Feststoffgehalt von mindestens 3 Massen-% und in einem molaren Äquivalentverhältnis zwischen dem Protein und der Anzahl der terminalen biotinylierten Enden der Polymerketten im Biotin-Polymer-Konjugat_von 1:2 bis 1:8 miteinander gemischt. Das Mischen der beiden Komponenten, vorzugsweise in einem Hochgeschwindigkeits-Wirbelmischer, verhindert, dass es anstelle der Hydrogelbildung zu einer Partikelformung kommt.

Der niedermolekulare Ligand Biotin beziehungsweise eines seiner Derivate, wie zum Beispiel Iminobiotin oder Desthiobiotin, wird an die Enden der Polymerkette konjugiert. Das Polymer kann ein Einzel- oder Doppelstrang-Oligonukleotid sein. Das Polymer kann auch eine verzweigte Oligonucleotid-Struktur sein, die mehr als zwei Enden enthält. Die resultierenden Polymer-Biotin-Konjugate werden durch ein tetrameres Protein vernetzt. Als tetrameres Protein können dabei Streptavidin oder Avidin oder Mutanten von Streptavidin und Avidin eingesetzt werden. Besonders bevorzugt ist dabei die Streptavidin-Mutante Traptavidin, die eine höhere Affinität zu Biotin als Streptavidin besitzt.

Die Biomaterial-Systeme können weiterhin entweder durch Einfügen von Peptidsequenzen in die Polymerkette oder durch Zugabe von biotinylierten Peptiden, biotinylierten Wirkstoffen, biotinylierten Oligosacchariden, biotinylierten Oligonukleotiden oder biotinylierten Proteinen in das Hydrogel modifiziert werden. Die Biotingruppe kann, wie bereits erwähnt, auch durch die Biotinanaloga Desthiobiotin oder Iminobiotin ersetzt werden, von denen erwartet wird, dass sie die Freisetzungsraten der Peptide, Wirkstoffe, Oligosaccharide, Oligonukleotide und Proteine erhöhen. Weiterhin können die oben erwähnten verschiedenen kovalenten oder nicht-kovalenten Modifikationen kombiniert werden, was eine unbegrenzte Anzahl von neuen Biomaterialien hervorbringt, welche für das Screening und die Optimierung von Biomaterialien durch einen kombinatorischen Ansatz verwendet werden können.

Ein weiterer Aspekt der Erfindung betrifft die Verwendung eines erfindungsgemäßen physikalischen selbstorganisierenden Hydrogelsystems für eine Zellkultur. Das heißt, die resultierenden Biomaterial-Systeme können zur Kultivierung von Zellen auf den Biomaterialien sowie für die Einkapselung von Zellen in das Hydrogel eingesetzt werden.

Weitere Einzelheiten, Merkmale und Vorteile der Erfindung ergeben sich aus der nachfolgenden Beschreibung von Ausführungsbeispielen mit Bezugnahme auf die zugehörigen Zeichnungen. Es zeigen:
- **Fig. 1:**: eine schematische Darstellung der kinetischen und thermodynamischen Kontrolle der Netzwerkbildung, kein Teil der Erfindung,
- **Fig. 2a:**: eine schematische Darstellung einer zweidimensionalen Hydrogelmatrix als eine Reihe von miteinander verbundenen Quadraten, kein Teil der Erfindung,
- **Fig. 2b:**: eine photographische Abbildung eines verbundenen Netzwerks, kein Teil der Erfindung,
- **Fig. 2c:**: eine photographische Abbildung eines verbundenen Netzwerks, kein Teil der Erfindung,
- **Fig. 3a:**: ein Diagramm mit einem Vergleich der relativen Gelhärte von Hydrogelen mit unterschiedlichem Feststoffgehalt, kein Teil der Erfindung,
- **Fig. 3b:**: ein Diagramm, das die Hydrogelerosion in Abhängigkeit vom Feststoffgehalt zeigt, kein Teil der Erfindung,
- **Fig. 3c:**: ein Diagramm, das die Hydrogelquellung in Abhängigkeit der lonenstärke zeigt, kein Teil der Erfindung,
- **Fig. 3d:**: ein Diagramm, das die Hydrogel-Haltbarkeit in Abhängigkeit von der lonenstärke zeigt, kein Teil der Erfindung,
- **Fig. 4:**: eine Größenausschluss-Chromatographie einer Hydrogellösung mit 1 Massen-% Feststoffgehalt, kein Teil der Erfindung,
- **Fig. 5a:**: eine Größenausschluss-Chromatographie für Hydrogellösungen nach der Erosion mit Neuordnung von Partikeln, kein Teil der Erfindung,
- **Fig. 5b:**: eine Größenausschluss-Chromatographie von erodiertem Hydrogel mit 9 Massen-% Feststoffgehalt nach einer Konzentrierung der Probe, kein Teil der Erfindung,
- **Fig. 6:**: eine photographische Abbildung eines Live-Dead-Assays, kein Teil der Erfindung,
- **Fig. 7a:**: eine mikroskopische Aufnahme einer typischen sich nicht ausbreitende Zelle, kein Teil der Erfindung,
- **Fig. 7b:**: eine mikroskopische Aufnahme einer teilweise sich ausbreitenden Zelle, kein Teil der Erfindung,
- **Fig. 7c:**: eine mikroskopische Aufnahme einer sich weit ausbreitenden Zelle, kein Teil der Erfindung,
- **Fig. 7d:**: hellfeldmikroskopische Aufnahme des 0 %-B-II-Hydrogels, kein Teil der Erfindung,
- **Fig. 7e:**: hellfeldmikroskopische Aufnahme des 50 %-B-II-Hydrogels, kein Teil der Erfindung,
- **Fig. 7f:**: hellfeldmikroskopische Aufnahme des 100 %-B-II-Hydrogels, kein Teil der Erfindung,
- **Fig. 7g:**: eine mikroskopische Aufnahme einer vollständigen Verteilung der Zellausbreitung, kein Teil der Erfindung, und
- **Fig. 8:**: ein Transliteration-Assay von stimulierten Maus T-Zellen unter Verwendung von Durchflusszytometrie in Abhängigkeit von CsA-azo-Biotin, kein Teil der Erfindung.

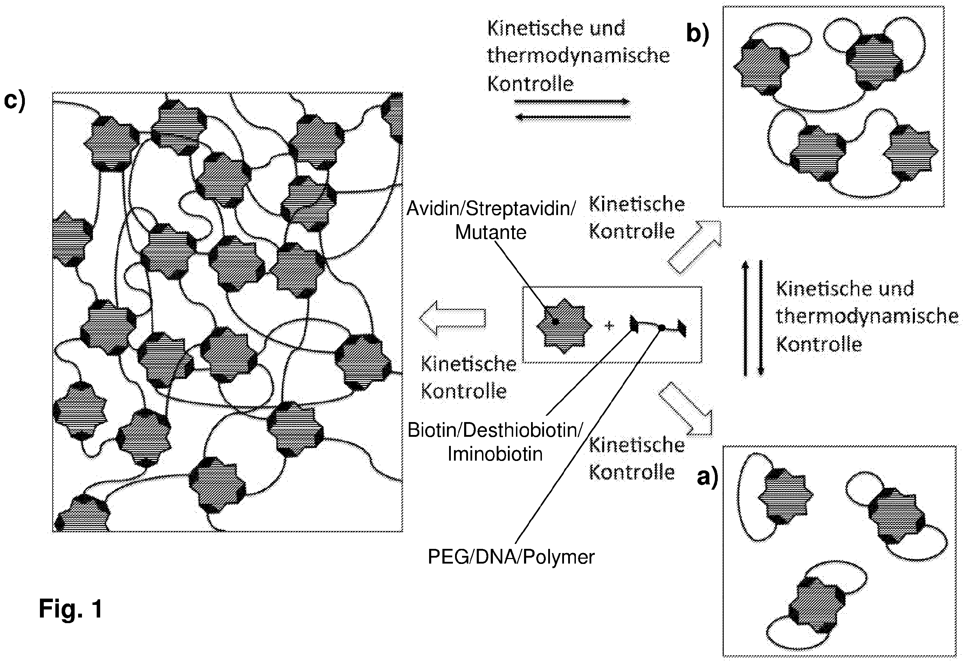

Die **Fig. 1** zeigt schematisch eine kinetisch-kontrollierte Hydrogelierung auf der Basis der Protein-Ligand-Wechselwirkung. Kinetische Kontrolle heißt: Abhängig vom Volumen bilden sich nur intramolekulare Verbindungen des PEG-Biotins zu einem Protein, zum Beispiel zu Avidin, Streptavidin oder einer Mutante dieser Proteine, bei niedriger Konzentration, siehe Abbildung **a**), kleine Partikel mit intra- und intermolekularen Verbindungen bei höherer Konzentration, siehe Abbildung **b**), oder Hydrogele mit fast ausschließlich intermolekularen Verbindungen bei höchster Konzentration, siehe Abbildung **c**). Kinetische und thermodynamische Kontrolle heißt: Da die Verbindungen sich in dynamischer Umlagerung, das heißt Kinetik, befinden, können die einzelnen in den Abbildungen **a, b ,c)** gezeigten "Stadien" durch Volumenänderung/Konzentrationsänderung ineinander umgewandelt werden. Auch erreicht das System immer (in jedem Stadium) das energetische und entropische Minimum, welches je nach Konzentration/Volumen in Partikelformation oder Hydrogelformation endet.

### Beispiel I: Streptavidin/Avidin-PEG-Biotin-Hydrogel, kein Teil der Erfindung

Aufgrund der zunehmenden Anwendung von nicht-kovalenten Hydrogelsystemen für biotechnologische Anwendungen wurde ein sich selbstorganisierendes Hydrogel synthetisiert, welches allein durch Rezeptor-Ligand-Wechselwirkung zwischen Biotin und Avidin vernetzt ist.

Dieses Modellsystem wurde genutzt, um das Verhalten von Rezeptor-Liganden auf der Basis von supramolekularen Netzwerken zu bestimmen und zu rationalisieren. Die Hydrogelbildung und die Erosion werden durch die physikalische Chemie von Rezeptor-Ligand-Wechselwirkungen bestimmt. Die Gel-Bildung ist einerseits kinetisch kontrolliert und tritt erst oberhalb einer kritischen Konzentration c* auf, andererseits kommt es aufgrund von kombinierten Effekten der Ligand-Umlagerung, Netzwerkquellung und Entropie schneller als erwartet zur Erosion. Eine Erosion tritt als fortschreitende Umlagerung des Gels in immer kleinere Partikel auf. Jedoch ist dieser Prozess konzentrationsabhängig und kann durch Konzentrierung der erodierten Hydrogel-Lösung umgekehrt werden. Schließlich wurden als Nachweis der Nützlichkeit eines solchen Systems Peptidmotive eingebaut, mit der Absicht, Stammzellen-Adhäsion und Ausbreitung zu fördern.

Viele Hydrogelsysteme wurden bisher auf der Basis von nicht-kovalenten Wechselwirkungen, wie zum Beispiel hydrophoben Wechselwirkungen, Protein-Protein- oder Peptid-Oligosacccharid-Wechselwirkungen, entwickelt.

Ein viel weniger genutzter Vernetzungsweg stellt demgegenüber die Nutzung einfacher Rezeptor-Ligand-Bindungen dar. Da die Netzwerkbildungen komplexe Prozesse sind, wurde bisher noch nicht gut verstanden, wie die Bindungskinetik und Bindungsenergie die Netzwerkbildung sowie die daraus resultierenden physikalischen, mechanischen und biochemischen Eigenschaften beeinflussen. Die schnelle Bindung und die Besonderheiten zum Beispiel der Biotin-Avidin-Bindung sind für eine Nanopartikelbildung sowie Hydrogelmodifikation und -funktionalisierung ausgenutzt worden, interessanterweise blieb aber die Verwendung als alleiniger Vernetzer in einem makroskopischen Netzwerk weitgehend unerforscht. Überraschenderweise wurde herausgefunden, dass dieses scheinbar einfache selbstorganisierende Netz nur unter fein abgestimmten Bedingungen gebildet werden kann. Obwohl die Biotin-Avidin-Bindung im Allgemeinen als fast irreversibel betrachtet wird, führt die physikalische Chemie der Protein-Ligand-Bindung und die Dissoziation zu einem dynamischen Netzwerk. Darüber hinaus wird das Netzwerk leicht funktionalisiert, um in diesem Fall die Morphologie von eingekapselten mesenchymalen Stammzellen in der 3D-Matrix zu beeinflussen.

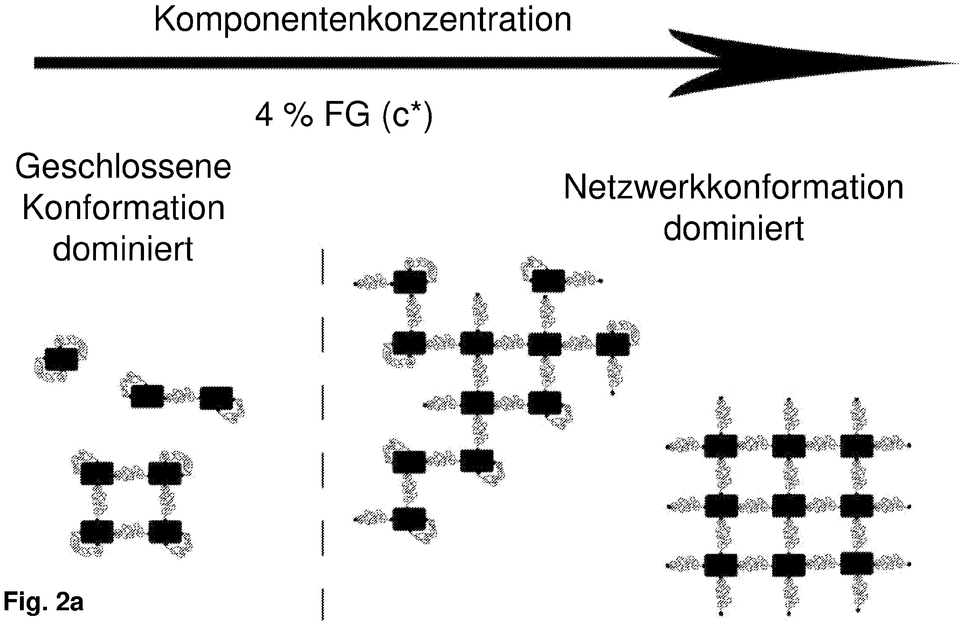

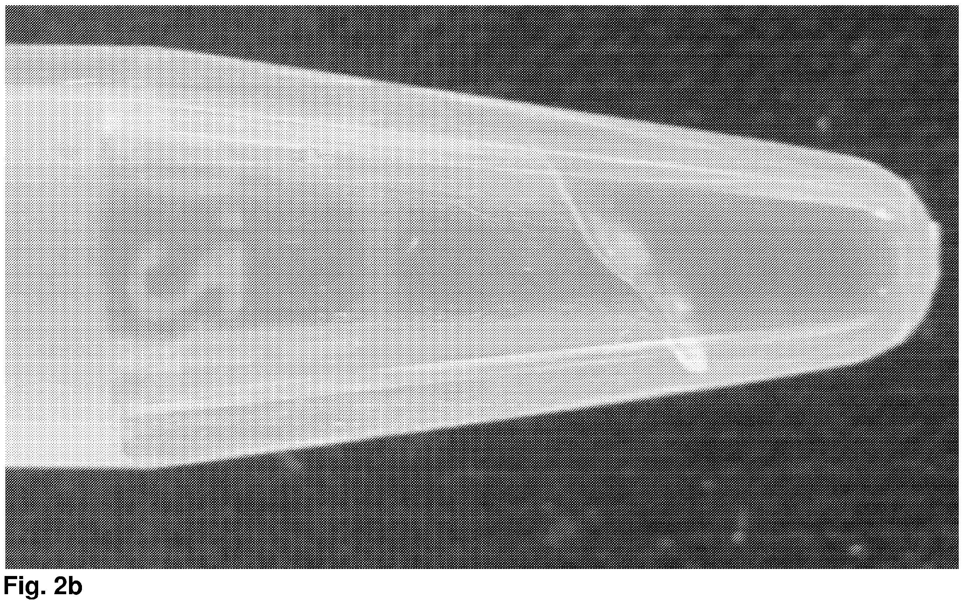

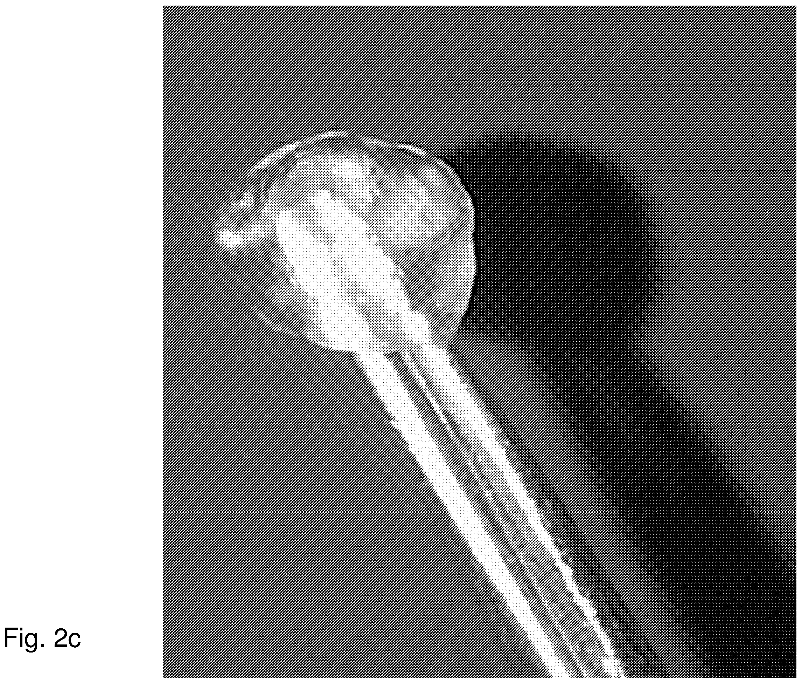

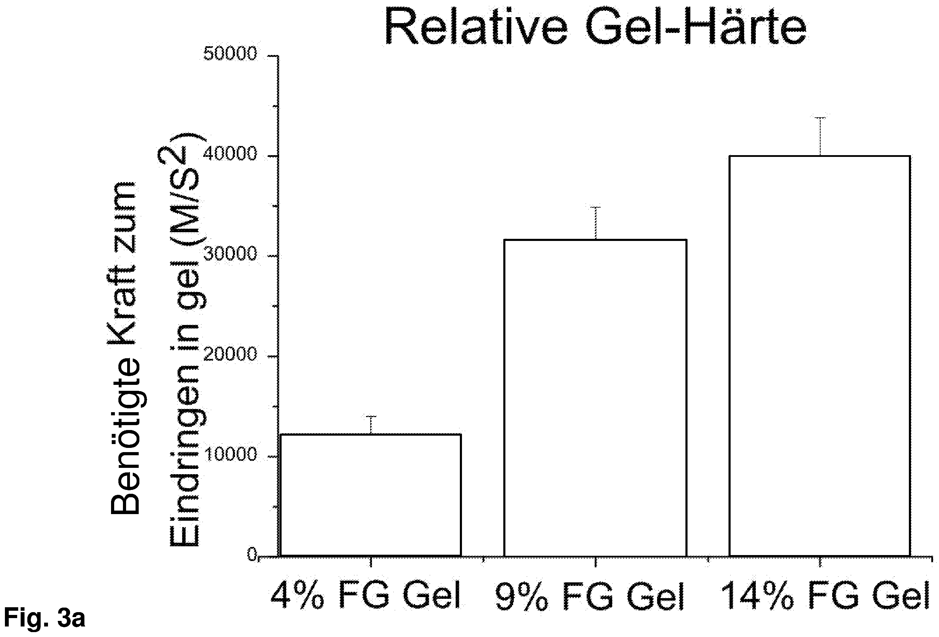

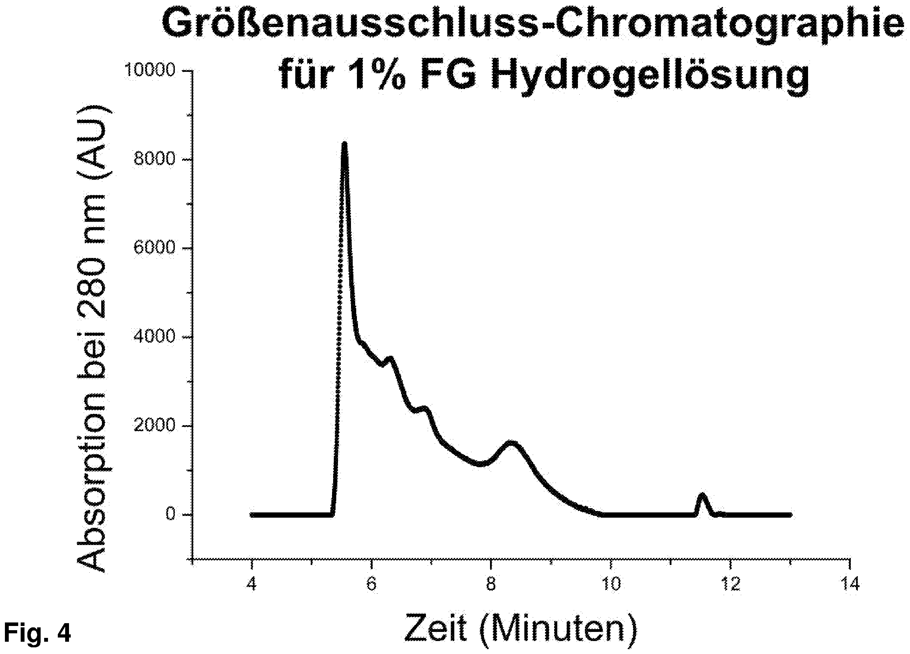

Die Hydrogel-Netzwerkbildung ist konzentrationsabhängig. In einem ideal selbstorganisierten Gitter dienen Avidin-Tetramere als Bindungszentrum, die durch an beiden Enden biotinylierte lineare Polyethylenglykol-(PEG)-Ketten vernetzt sind. Die strukturelle Einfachheit des Hydrogels ermöglicht es, die Matrix zweidimensional als eine Reihe von miteinander verbundenen Quadraten zu modellieren, wie die **Figur 2a** zeigt. Durch die steile Biotin-Avidin-Bindungs-Konstante Kₒₙ erlangt die Komponentenkonzentration entscheidende Bedeutung für die Netzwerkbildung. Für die Bildung eines vollständigen Netzwerks müssen die Zwischenräume zwischen den Avidin-Bindungsstellen ähnlich oder kleiner als der theoretische Durchschnittsabstand der Enden der biotinylierten PEG-Linker zueinander sein. Wenn die Topologie geeignet ist, kann eine PEG-Kette zwei verschiedene Avidin-Tetramere binden, was in der sogenannten "Netzwerk-Konformation" erfüllt ist. Je mehr sich die Konzentrationen verringern und die Abstände größer werden, desto mehr erhöht sich die Wahrscheinlichkeit, dass eine PEG-Kette sich an das gleiche Avidintetramer in einer sogenannten "geschlossenen Konformation" zweimal bindet, so dass das erste Bindungsereignis den Bereich beschränkt, in dem die PEG-Kette eine andere Bindungsstelle suchen kann. Daher wird sich ein fragmentiertes, unvollständiges Netz bilden, das aus einem Gemisch von Netzwerkbestandteilen und geschlossenen Konformationsteilchen besteht, wenn die Zwischenräume für das PEG auch nur geringfügig zu groß sind, um die Abstände zu überbrücken. Wenn die Räume für das PEG viel zu groß sind, um die Abstände zu überbrücken, wird sich kein zusammenhängendes Netz bilden, sondern eine Verteilung von Mikro- und Nano-Gelteilchen mit geschlossenen Konformationsrändern. Unter der Annahme, dass die PEG 10 kDa-Kette in ihre Konturlänge von 33,2 nm gestreckt wird, könnte sich ein Hydrogel mit einem Feststoffgehalt von 0,21 Massen-% (27 µM Avidin und 54 µM Biotinyl-PEG) bilden. Jedoch wird unter diesen Bedingungen kein Hydrogel gebildet, da die freien Polymere energetisch eine halbgewendelte Random-Coil-Struktur bevorzugen. Es wurde der mittlere Abstand der Enden einer 10 kDa-PEG-Kette durch die Modellierung des Polymers unter Anwendung des "Modells einer wurmartigen Kette" berechnet. Die Berechnungen zeigen einen mittleren End-End-Abstand von 5,0 nm für eine 10 kDa-PEG-Kette. Unter der Annahme einer idealen Verteilung von Avidin in Lösung wurde berechnet, dass ein Hydrogel-Feststoffgehalt von 9 Massen-%, das entspricht 1,27 mM Avidin, Lücken von 5,1 nm zwischen den Avidin-Tetrameren ergeben und damit in etwa dem Mindestfeststoffgehalt entsprechen würde, der erforderlich ist, um ein verbindendes Netzwerk zu bilden, wie es in den **Figuren 2b** und **2c** gezeigt ist. Obwohl Hydrogele mit einem Feststoffgehalt gebildet werden können, der so niedrig ist wie 4 Massen-%, sind sie aufgrund des Patchwork-Charakters des unvollständigen Netzwerks deutlich weicher, wie die **Fig. 3a** zeigt, und weniger stabil als Gele mit höherem Feststoffgehalt. Mischungen mit einem Feststoffgehalt von unter 4 Massen-% bilden kein zusammenhängendes Netzwerk. Dabei zeigt die Größenausschluss-Chromatographie in **Fig. 4** für eine Gel-Lösung mit 1 Massen-% Feststoffgehalt eine Streuung von kleinen Gel-Teilchen.

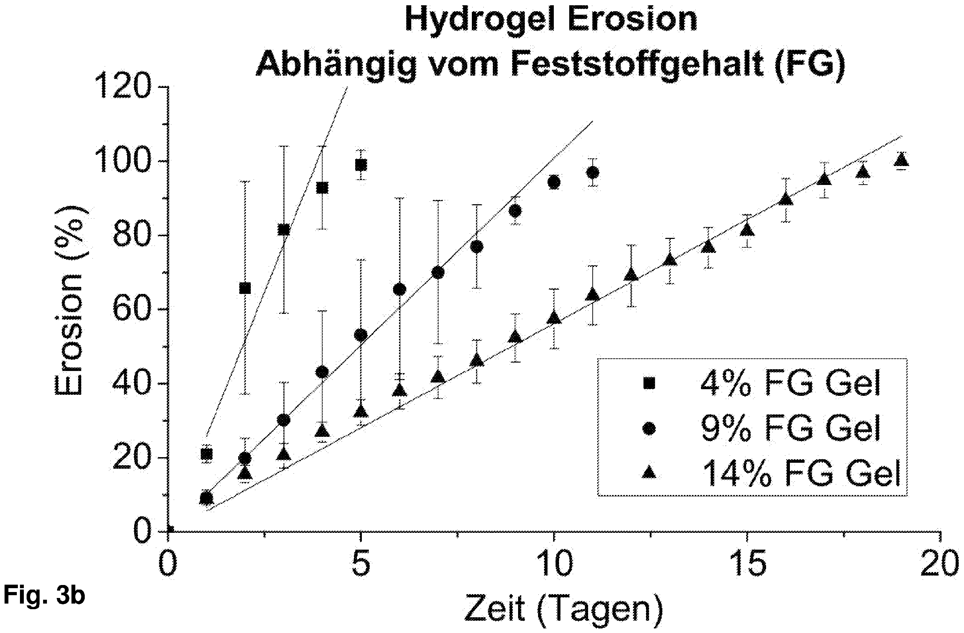

Die Hydrogel-Erosion ist ein linearer und konzentrationsabhängiger Prozess. Da die Biotin-Avidin-Wechselwirkung generell im Hinblick auf die Stabilität ähnlich einer kovalenten Bindung betrachtet wird, war es überraschend, dass die Erosion der Hydrogele im Überstand erst in einer Zeitskala von Tagen bis Wochen auftrat, während die Hydrogele in Abwesenheit von Überstand über Monate stabil blieben, wie in **Fig. 3b** zu sehen. Die Hydrogel-Stabilität konnte durch Einstellen des Feststoffgehalts von einer Lebensdauer von vier Tagen bei 4 Massen-% Feststoffgehalt auf eine Lebensdauer von 20 Tagen bei 14 Massen-% Feststoffgehalt angepasst werden. In allen Fällen erodieren die Hydrogele linear, was typisch für physikalische Hydrogele ist. Wie die vorhergehende Darstellung der verschiedenen möglichen Konformationen der Biotinyl-PEG/Avidin-Wechselwirkungen zeigt, ist es auch möglich, den physikalischen Mechanismus für die Erosion zu beschreiben. Wenn eine Biotin-Avidin-Bindung aus einer Netzwerk-Konformation herausbricht, kann das PEG-Biotinyl-Konjugat entweder ein Avidin in einer Netzwerk-Konformation oder eine andere verfügbare Stelle des gleichen Tetramers binden, indem es sich in einer geschlossenen Konformation platziert, was zur Erosion durch Diffusion aus einem Gel-Körper führt. Unter der Annahme, dass die Bindungsenergie zwischen Avidin und Biotin in jeder Netzwerk-Konformationen gleich ist, erhöht sich der Unterschied zwischen der Entropie des erodierten Zustands und der Gitterstruktur, wie sich das Volumen des gesamten Systems durch Zugabe von Pufferlösung auf das Hydrogel erhöht. Daher ist einerseits die Erosion der geordneten Struktur im Überstand thermodynamisch begünstigt. Andererseits wird die Kinetik der strukturellen Umlagerung auch von der Hydrogel-Quellung sowie der Dissoziation von Biotin aus Avidin beeinflusst.

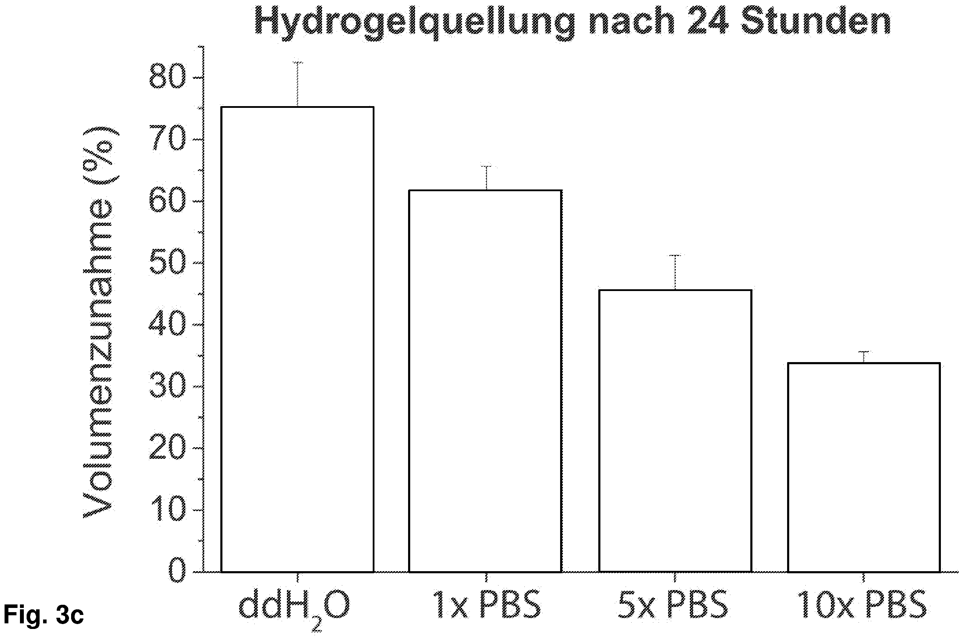

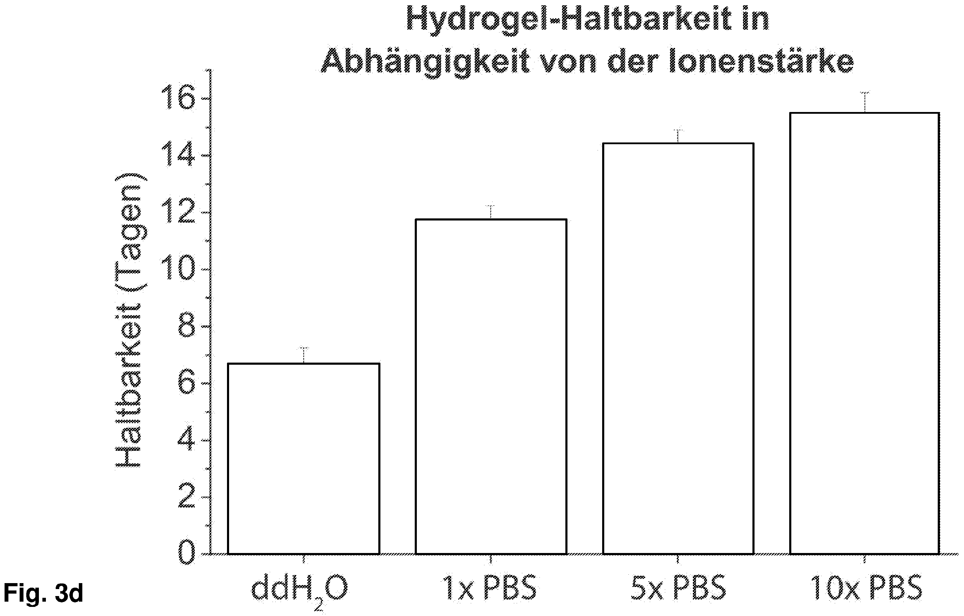

Des Weiteren beeinflusst eine Quellung die Hydrogel-Lebensdauer. Das Biotinyl-PEG/Avidin-Hydrogel unterläuft eine Quellung, wenn es im Überstand platziert ist. Die Quellung wird durch den osmotischen Druck - aufgrund der größeren Konzentration der gelösten Stoffe im Inneren des Hydrogels als außen - angetrieben und endet, wenn entweder das osmotische Gleichgewicht erreicht ist oder die elastischen Kräfte aus der Ausdehnung der HydrogelKomponente dem osmotischen Druck widerstehen können. Um die Auswirkungen der Quellung des Hydrogels zu untersuchen, wurde die lonenstärke des Hydrogels und des Überstands eingestellt. So wurde die Hydrogelquellung in deionisiertem Wasser (ddH2O) der Hydrogelquellung in verschiedenen Phosphat-gepufferten Kochsalzlösungen (PBS) in jeweils unterschiedlicher Konzentration, das heißt 1x PBS, 5x PBS und 10x PBS, gegenübergestellt. Wie die **Fig. 3c** zeigt, ist es durch Überführen des Hydrogels aus deionisiertem Wasser (Milli-Q®-Wasser, ddH₂O) in 10x Phosphat-gepufferte Kochsalzlösung (PBS) möglich, den Betrag der in einem Hydrogel mit 9 massen-%-igem Feststoffgehalt und in einem Zeitraum von 24 Stunden auftretenden Quellung von ∼ 74 % auf 35 % zu reduzieren. Interessanterweise haben die Hydrogele mit reduzierter Quellung eine längere Lebensdauer als die Vergleichsbeispiele mit erhöhter Quellung, wie ein Vergleich mit **Fig. 3d** zeigt. Offenbar trägt die Hydrogel-Quellung in zweierlei Hinsicht zur Erosion bei. Zunächst dehnen sich die PEG-Ketten durch die Quellung über ihre Gleichgewichtslänge aus. Wenn eine Biotin-Avidin-Bindung gebrochen ist, wird sich das PEG in seine Gleichgewichtslänge zurückzuziehen und ist somit eher geeignet, in einer geschlossenen Konformation Bindungen einzugehen als ein Netzwerk neuzubilden.

Des Weiteren setzt die Quellung die Ketten durch die Ausdehnung des Polymers über seine Gleichgewichtslänge hinaus unter Spannung. Das Hydrogel mit 9 Massen-% Feststoffgehalt quillt in 1x PBS innerhalb von 24 Stunden 60 Vol.-%, wobei sich das PEG von 5,0 nm auf 5,8 nm ausdehnt. Bei erneuter Betrachtung des Polymers als wurmartige Kette, wurde die wirkende Spannung aufgrund der Ausdehnung des Polymers mit 3,2 pN berechnet. Die Belastungsgeschwindigkeit betrug etwa 0,32 pN pro Stunde. Ungeachtet der Biotin-Avidin-Bruchfestigkeit, die häufig als im Bereich von Hunderten von pN liegend genannt wird, werden die genannten Werte hoher Belastungsgeschwindigkeit erzielt. Berechnungen zufolge könnte bei Vorliegen einer sehr kleinen Belastungsgeschwindigkeit die Biotin-Avidin-Bindung durch nur ∼ 3 pN direkt aufgebrachter Kraft gebrochen werden, während Messungen mit einer optischen Falle bei Belastungsgeschwindigkeiten von 7,7 pN pro Sekunde einen Bruch bei so niedrigen Kräften wie 3,4 pN anzeigten. Während die feste Bindung bei hoher Belastungsgeschwindigkeit für die verschiedenen Anwendungen von Biotin-Avidin in der Biotechnologie von höchster Bedeutung ist, wird interessanterweise im Hydrogel-Erosionsexperiment die schwache Wechselwirkung des Bindungspaares, wenn es bei niedriger Belastungsgeschwindigkeit gestreckt wird, veranschaulicht. Das Biotin-Avidin-Hydrogel ist bei hoher mechanischer Beanspruchung wie bei UltraschallBehandlung stabil, während die physikalische Belastung, die sich aus dem langsamen Quellungsprozess ergibt, die Interaktion zwischen diesem ansonsten sehr starken Rezeptor-Ligand-Paar stören kann.

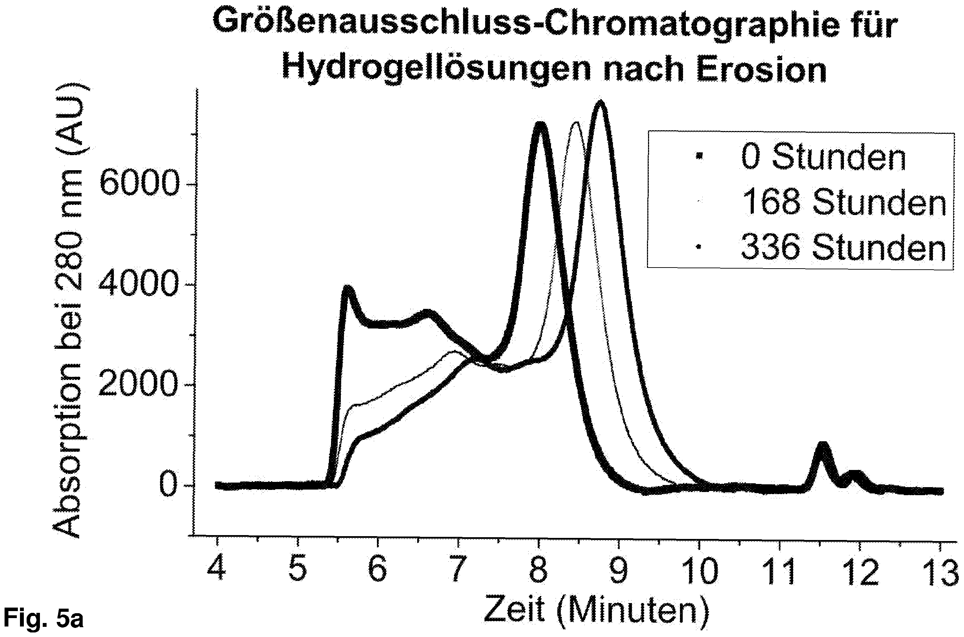

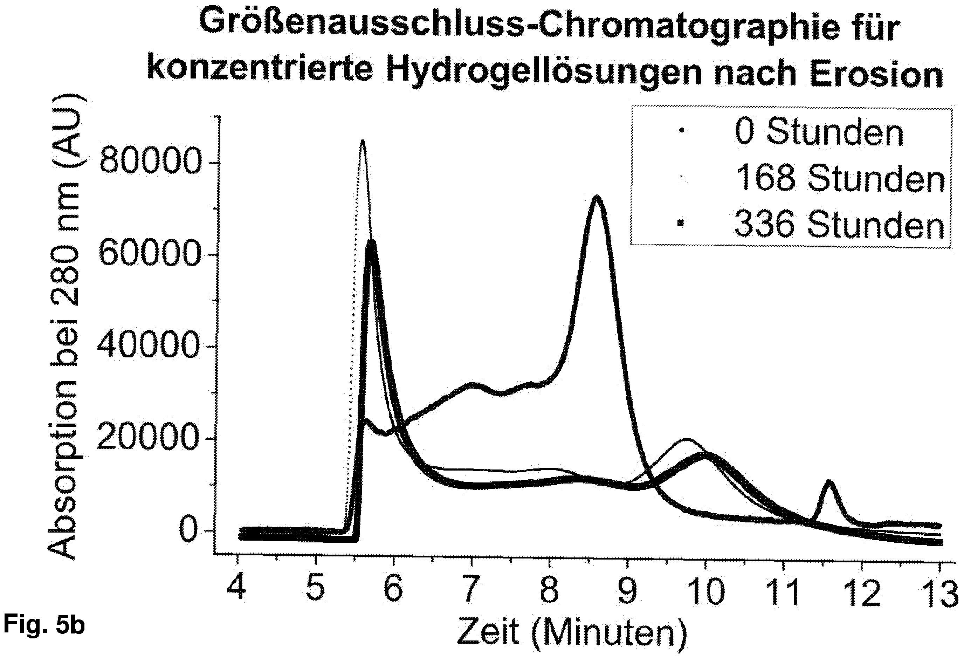

Die Erosion ist fortschreitend und reversibel. Während die Hydrogel-Erosion ein thermodynamisch begünstigter Prozess der geschlossenen KonformationsTeilchen ist, die aus dem Hydrogel in den Überstand diffundieren, konnte für die erodierten Hydrogel-Lösungen mittels der Größenausschluss-Chromatographie festgestellt werden, dass auch dann, wenn ein zusammenhängendes Hydrogel verschwunden ist, sich die Partikel in Lösung über die Zeit weiterhin in kleineren Fragmenten neu ordnen, wie auch aus der **Fig. 5a** hervorgeht. Es ist von außerordentlicher Bedeutung, dass die Gelierungs-/Erosionsprozesse reversibel sind. Die erodierten Hydrogel-Lösungen wurden in ihre ursprünglichen Volumina zurück konzentriert, wobei sich herausstellte, dass Hydrogele über einen Zeitraum von 72 Stunden neu gebildet wurden. Dies ist ein relativ langer Zeitraum im Vergleich zu der nahezu sofortigen Bildung der ursprünglichen Hydrogele. Zur weiteren Untersuchung dieses Prozesses wurde 1 ml Lösung von 30 µl erodiertem Hydrogel mit 9 Massen-% Feststoffgehalt zu 100 µl konzentriert, was zu einem Feststoffgehalt etwas unterhalb des kritischen Feststoffgehalts für die Hydrogel-Bildung führt, und eine Größenausschluss-Chromatographie durchgeführt. Deren Ergebnis ist in der **Fig. 5b** gezeigt. Unmittelbar vor dem Konzentrieren der Probe war der Hauptpeak bei 8 ± 0,02 Minuten eluiert. Nach einer Zeit von 168 und 336 Stunden nach der Konzentrierung der Probe wurde der Hauptpeak mit den Einspritzpeaks eluiert. Diese Daten zeigen, dass die Netzwerkbildung bei hoher Konzentration begünstigt ist.

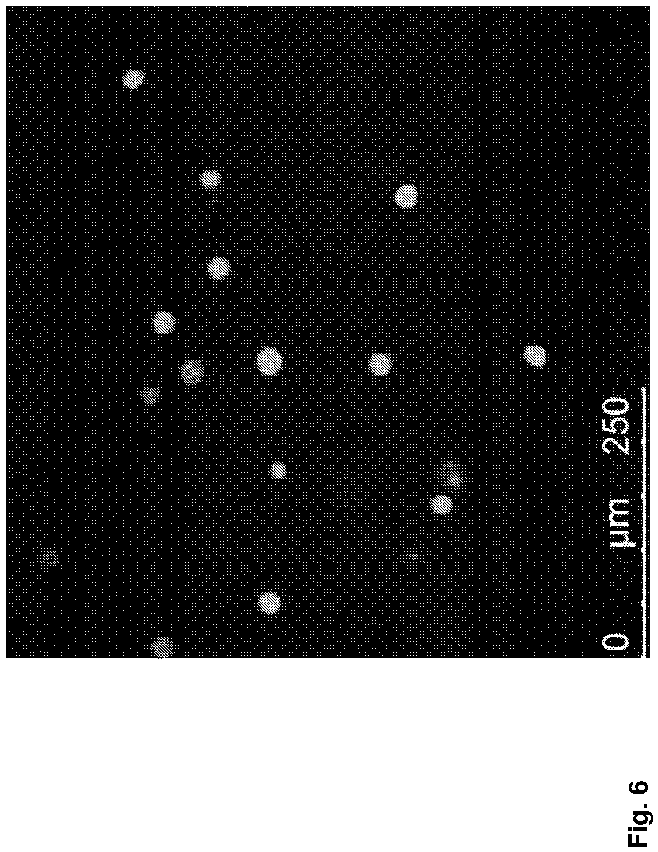

Hydrogele sind biokompatibel und geeignet zur 3D-Stammzellenkultur. Ein sich schnell selbstorganisierendes Hydrogel ist das Mittel der Wahl für das Screening optimaler Bedingungen für 3D-Zellkultur-Anwendungen. Es wurde untersucht, ob die Zellen in der Lage sein würden, in den 3D-Matrices zu überleben und sich auszubreiten. Es wurden modifizierte PEGs präpariert, die Biotin-Peptid-Konjugate enthielten. Das erste Konjugat Biotin-GRGDSPGWC, im Folgenden BI bezeichnet, enthielt nur das Zelladhäsions-Motiv RGDSP. Das zweite Konjugat Biotin-GRGDSPQGIWGQC, im Folgenden B-II bezeichnet, enthielt sowohl das Zelladhäsions-Motiv als auch die Spaltstelle von Matrix-Metalloproteinase (MMP), PQG ↑ ↓ IWGQ, so dass die Zellen chemisch das Netzwerk spalteten. Die Hydrogel-Komponenten-Lösungen wurden mit humanen mesenchymalen Stammzellen (MSCs) gemischt, was zu eingekapselten Zellen in einem selbstorganisierenden Netzwerk führt. Nach 24 Stunden Inkubation wurden die Hydrogele mit einem Live/Dead-Assay getestet, welches 98,10 % überlebende Zellen zeigte (n = 157)**(siehe** **Fig. 6****).**

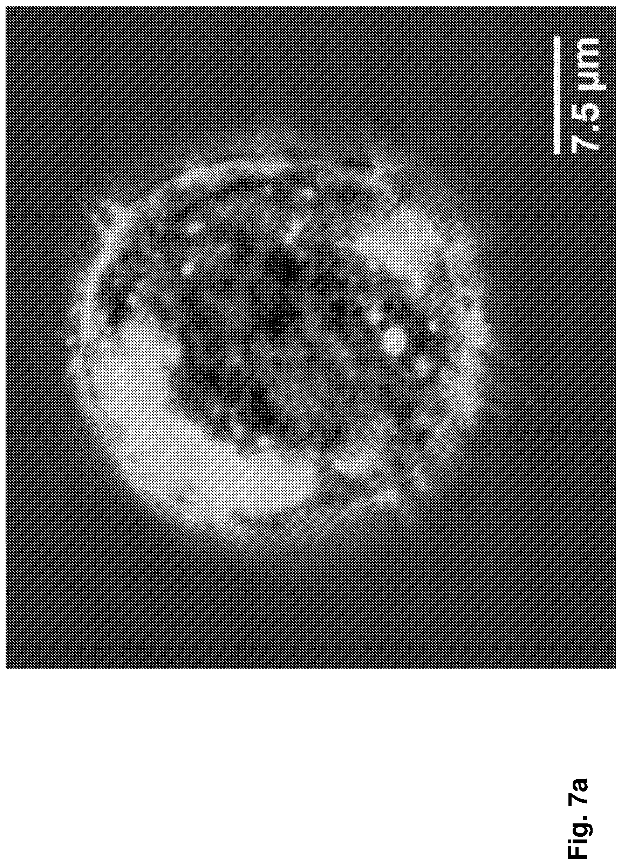

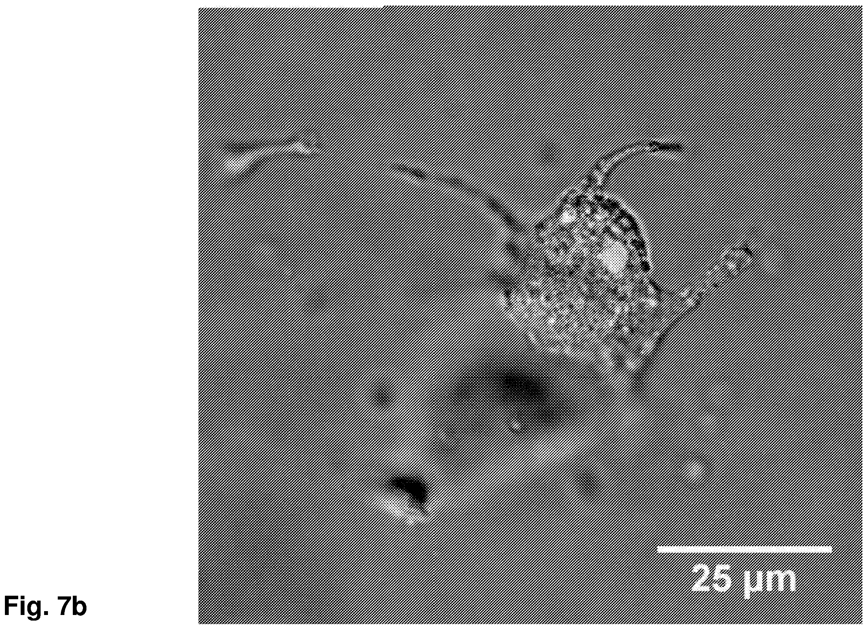

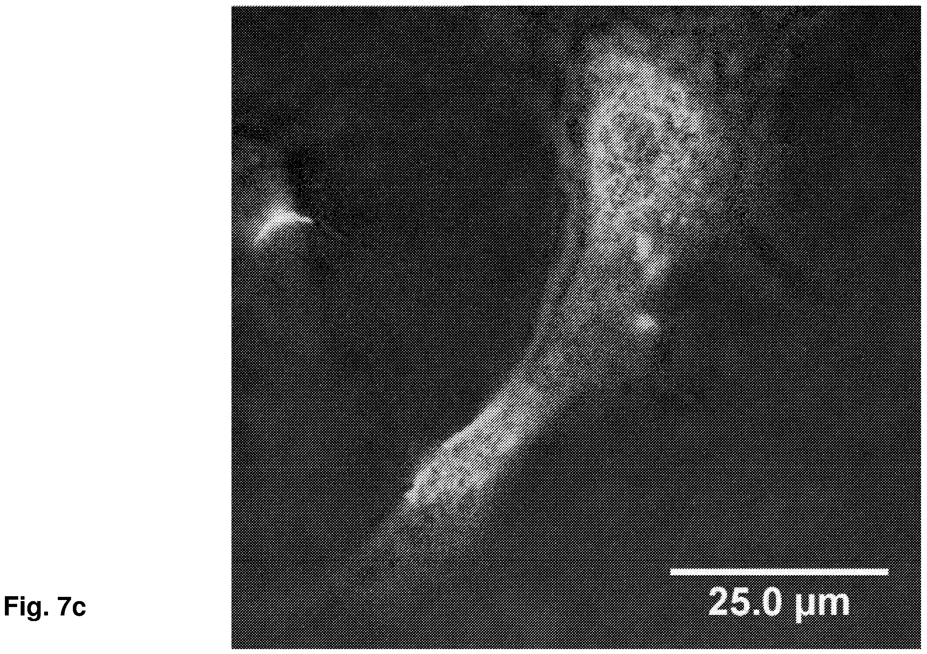

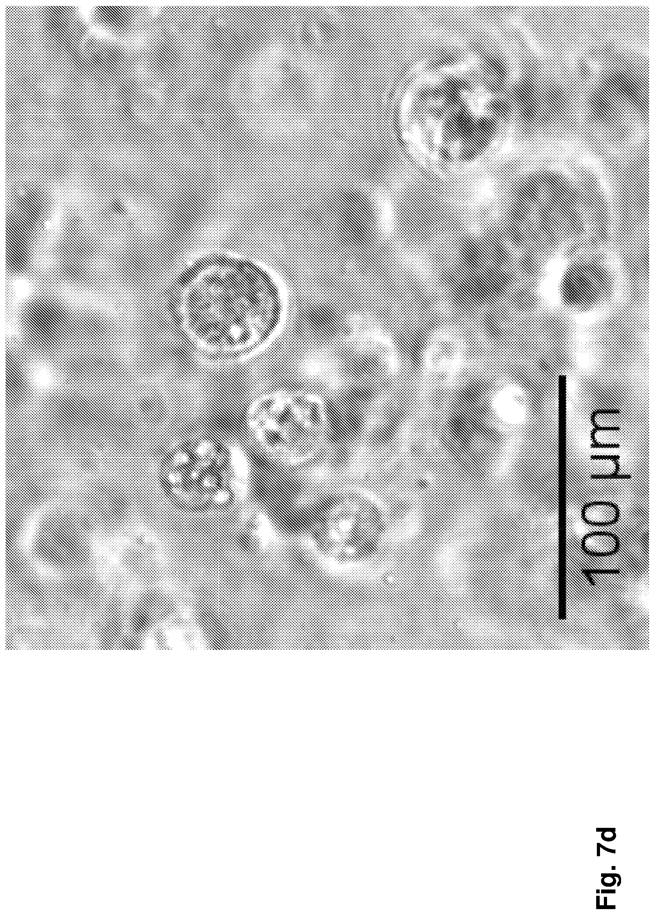

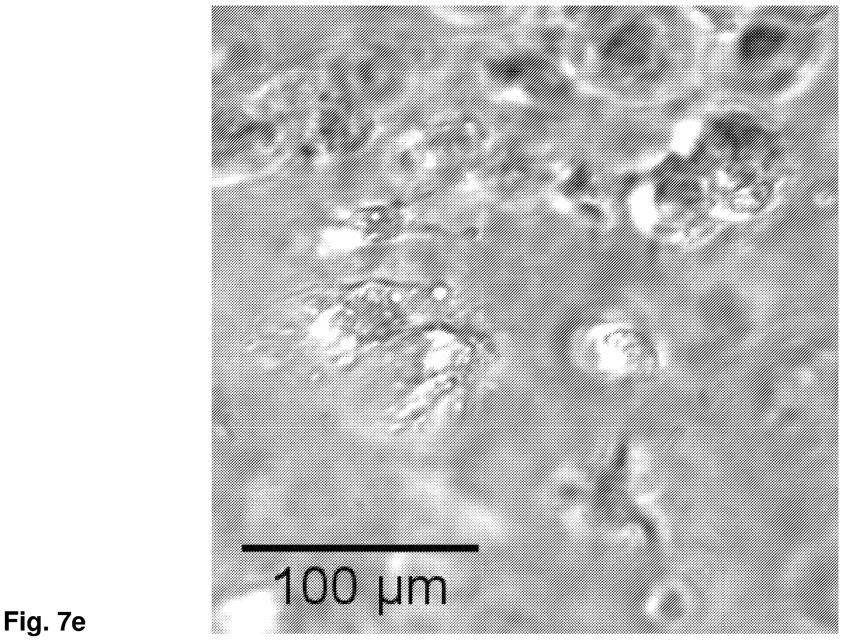

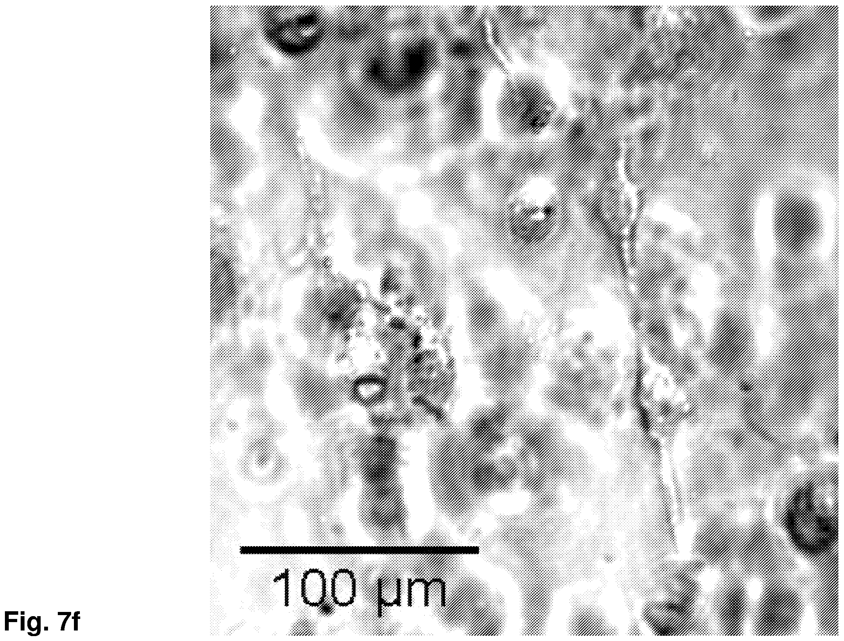

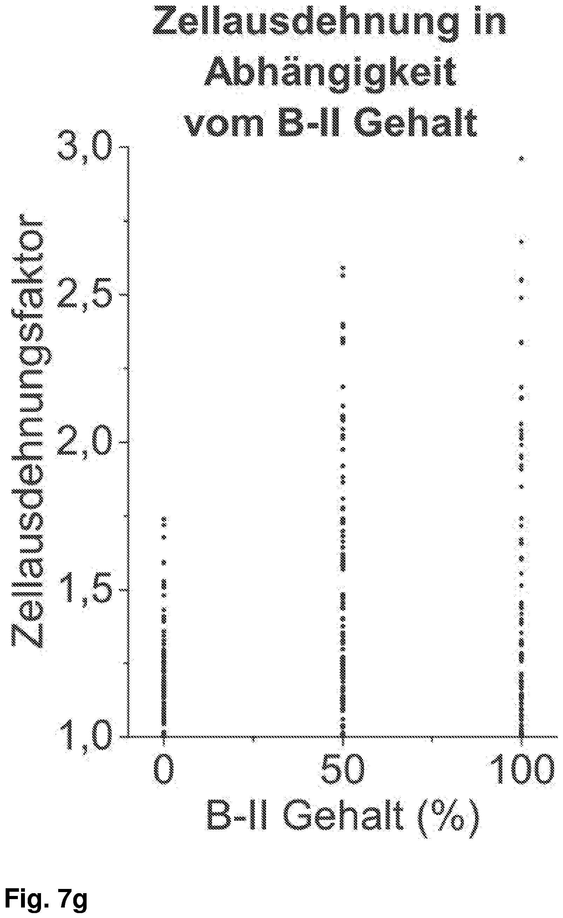

Als Nächstes wurde untersucht, ob die Hydrogele Zellausbreitung und Wachstum ermöglichen. Es wurden zunächst BI und B-II gemischt, das heißt eine Vormischung hergestellt, um Hydrogele mit 0 %, 50 % und 100% B-II herzustellen. Eine typische immune, sich nicht ausbreitende Zelle ist in **Fig. 7a** gezeigt. Eine teilweise sich ausbreitende Zelle ist in **Fig. 7b** abgebildet. Eine sich weit ausbreitende Zelle ist in **Fig. 7c** dargestellt. Repräsentative hellfeldmikroskopische Aufnahmen der 0 %-, 50 %-, 100 %-B-II-Hydrogele werden jeweils in den **Figuren 7d****,** **7e** und **7f** gezeigt. Eine vollständige Verteilung der Zellausbreitung ist in **Fig. 7g** gegeben. Nach 48 Stunden zeigt im 0 %-Hydrogel ein Anteil von 6,55 % der Zellen eine Ausbreitung an (n = 121), während im 5 %- Hydrogel ein Anteil von 31,95 % der Zellen (n = 131) und im 100 % -Hydrogel ein Anteil von 25,15 % der Zellen eine Ausbreitung (n = 130) anzeigen. Das Biotinyl-PEG/Avidin-Hydrogelsystem ist biokompatibel und für die Zellkulturanwendungen mit der MMP-spaltbaren Sequenz in B-II geeignet, die für die MSC-Ausbreitung erforderlich ist.

Vorliegend wurde ein neues, nicht-kovalentes Hydrogel bereitgestellt, welches durch die Rezeptor-Ligand-Wechselwirkung zwischen Biotin und Avidin vernetzt ist. Das bemerkenswerteste Merkmal dieses Hydrogels ist, dass die Hydrogel-Bildung und Erosion durch die physikalische Chemie der Rezeptor-Ligand-Wechselwirkungen bestimmt wird. Einerseits ist die Hydrogel-Bildung ein kinetisch gesteuerter Prozess und tritt erst oberhalb einer kritischen Konzentration auf, andererseits führen die Streckspannung der Polymerketten sowie eine erhöhte Wahrscheinlichkeit, dass nach Brechung der Bindung während der Ausbreitung des Hydrogels ein PEG eine geschlossene Bindungskonformation annimmt, im Ergebnis zu einer Erosion des Hydrogels. Ebenfalls interessant war die Beobachtung, dass eine Erosion über eine fortschreitende Umlagerung der Hydrogelpartikel in eine zunehmende Zahl von Teilchen mit kleineren Kernbereichen erfolgt. Darüber hinaus ist diese Umlagerung bidirektional und kann über die Konzentrierung des erodierten Hydrogels umgekehrt werden. Das Hydrogel-System zeigt, dass Netzwerke, die auf Rezeptor-Ligand-Wechselwirkungen basieren, geeignete Wege für die Entwicklung von neuen Biomaterialien sind, welche interessante Eigenschaften besitzen. Die Hydrogele organisieren sich unter milden Bedingungen selbst und weisen lineare, einstellbare Erosionsraten auf. Die Bedingungen, um eine 3D-Stammzellkultur zu fördern, könnten durch die Erforschung der Synergieeffekte der verschiedenen Peptidsequenzen abgestimmt werden. Da die Biotinylierung die am weitesten verbreitete Methode ist, um Biomoleküle zu immobilisieren, stellt das neuartige System einen einfachen Ansatz dar, hoch funktionalisierte Bio-Matrices zu entwerfen und zu überwachen.

Im Folgenden werden die angewendeten Methoden vorgestellt.

### Präparation der Hydrogelkomponente:

Avidin (Affiland, Belgien) wurde als lyophilisiertes Pulver erhalten. Biotinyliertes PEG wurde durch Auflösen von Biotin-NHS- und amino-terminiertes 10 kDa-PEG (Rapp Polymere, Deutschland) in einem Molverhältnis von 12:1 in Milli-Q-Wasser mit einem auf pH 9,5 eingestellten pH-Wert präpariert. Die Gesamtkonzentration betrug 31 mg/mL. Die Lösung wurde über Nacht gerührt. Das Reaktionsgemisch wurde dann zwei Tage lang gegen destilliertes Wasser dialysiert (8 kDa MW cutoff), um nicht-verbundenes Biotin zu entfernen. Die Lösung wurde dann lyophilisiert.

### Hydrogelbildung:

Um die Hydrogele zu bilden, wurden Biotinyl-PEG und Avidin in Lösungen gleichen Volumens in einem molaren Verhältnis von 2:1 in der passenden Konzentration für den gewünschten Feststoffgehalt gelöst. Es wurden 15 µL Avidin-Lösung in ein Mikrozentrifugenröhrchen pipettiert und in einen Hochgeschwindigkeits-Wirbelmischer gegeben. Unter Mischung wurden 15 µL Biotinyl-PEG-Lösung zugegeben, was zu einer nahezu sofortigen Hydrogelbildung führte.

### Hydrogel-Steifigkeit:

Eine Tischzentrifuge wurde verwendet (5424R, Eppendorf, Hamburg, Deutschland), um kleine Mengen von Hydrogel in einem Hochdurchsatzverfahren zu vergleichen. Es wurden 30 µL Hydrogele in 1x PBS hergestellt. Die relative Steifigkeit der Hydrogele wurden mittels 45 °-Zentrifugenrotor und Eindringen von 275 µm-Metallkügelchen in Abhängigkeit von der durch die Zentrifuge angelegten Kraft analysiert.

### Hydrogel-Erosion:

Bei der Untersuchung der Hydrogel-Erosion wurden die Gele, wie zuvor beschrieben, mit dem Zusatz von 10 µL (0,18 nmol) Cyanin-5-markiertem Streptavidin (Invitrogen) gebildet. Cyanin 5, im Folgenden Cy5 genannt, ist ein blauer Fluoreszenzfarbstoff. Nach der Hydrogelbildung wurde 1 ml Überstand vorsichtig auf die Oberseite jedes Hydrogels hinzugegeben. Die Gele wurden dann in einem Rotationsmischer mit 5 Umdrehungen pro Minute platziert und vor Licht geschützt. Aller 24 Stunden wurden 125 µl Überstand aus jedem Hydrogel entnommen und die Fluoreszenz-Aktivität an einem Synergy H1-Plate-Reader bei 645 nm Emission und 675 nm Anregung gemessen. Der entnommene Überstand wurde dann mit 125 µl frischem Überstand ersetzt, und die Fluoreszenz-Daten wurden mit einer Standardkurve verglichen, um die Erosion zu bestimmen.

### Live/Dead-Assay:

Die Lebensfähigkeit der Zellen in den PEG-Hydrogelen wurde mittels eines Live/Dead-Assays untersucht. Zellen enthaltende Gele wurden einmal mit PBS gespült. Eine Lösung von 10 µM Propidiumjodid (PI) (Molecular Probes, Invitrogen, Deutschland) und 0,15 µM Fluorescein-Diacetat (FDA) (Fluka, Deutschland) in PBS wurde 3 min lang auf die Gele aufgebracht, gefolgt von einem Waschschritt mit PBS. Zellen wurden mit einem konfokalen Mikroskop (Leica SP5 , 10x / 0,4) aufgenommen. Aufnahmen wurden alle 10 µm für einen Gelabschnitt von 100 µm Dicke entnommen und die Projektion der maximalen Intensität der Aufnahmen präsentiert. Helligkeit und Kontrast wurden in Fiji-Build von ImageJ angepasst.

### Zellausbreitungsmessung:

Es wurden 48 Stunden nach der Gel-Bildung Bilder von verschiedenen z-Ebenen der Hydrogele aufgenommen. Einzelne Zellen wurden identifiziert und mit Ellipsen unter Nutzung von Fiji-Build von ImageJ ausgestattet. Die Excentrizitäten der gemessenen Ellipsen wurden verwendet, um die Ausbreitung zu bewerten. MSCs mit Exzentrizitäten > 1,5 werden als Ausbreitung klassifiziert.

### Beispiel II: Traptavidin-PEG-Biotin-Hydrogel, kein Teil der Erfindung

Da die Erosion des Avidin/Streptavidin-Hydrogels durch die Dissoziation von Biotin aus tetrameren Proteinen ausgelöst und von der Netzwerk-Umlagerung gefolgt wird, wodurch sich die Dissoziationsgeschwindigkeit verringert, ist zu erwarten, dass die Matrix-Stabilität verbessert wird. Während die Avidin/ Streptavidin-Biotin-Wechselwirkung eine der höchsten Affinitäten zwischen einem Protein und einem Liganden in der Natur besitzt, wurde für die Streptavidin-Mutante Traptavidin vor kurzem berichtet, dass sie eine 10-mal langsamere Biotin-Dissoziationsgeschwindigkeit hervorbringt.

In diesem Beispiel wurden die vorteilhaften biophysikalischen Eigenschaften von Traptavidin genutzt, um die Hydrogel-Stabilität zu verbessern. Das Protein wurde in *E. coli* exprimiert und das gereinigte Protein zur Bildung des Hydrogels mit PEG-Biotin eingesetzt. Wie erwartet, konnte das selbstorganisierte Hydrogel wie in dem Avidin-PEG-Biotin-System nur oberhalb der kritischen Konzentration von 4 Massen-% Feststoffgehalt gebildet werden. Des Weiteren blieben die Hydrogele ohne Zugabe von Überstand über einen Zeitraum von mehreren Monaten stabil.

Wenn die Traptavidin-PEG-Biotin-Hydrogele mit dem Überstand inkubiert wurden, trat in einem Zeitraum von zwei Monaten keine sichtbare Erosion auf. Es wurde 1 % Cy5-markiertes Streptavidin zu nicht-markiertem Traptavidin zugesetzt, um das Hydrogel zu bilden. In dem Avidin-Hydrogel-System korrelierte die Freisetzung von Cy5-markiertem Streptavidin gut mit dem Erosionsprozess und dem Verlust an Hydrogelmasse, während Cy5-markiertes Traptavidin während einer Zeitdauer von 2 Monaten nachgewiesen werden konnte. Im Ergebnis demonstrierte dieses Experiment, dass die langsame Dissoziationsrate zwischen Traptavidin und Biotin überraschenderweise eine drastische Zunahme der Stabilität des selbstorganisierten Matrix-Netzwerks bewirkt.

### Beispiel III: Streptavidin/Avidin/Traptavidin-DNA-Biotin-Hydrogel, Ausführungsbeispiel der Erfindung

Das Hydrogel kann auch durch Ersetzen des biotinylierten PEG-Linkers mit biotinylierten doppelsträngigen DNA hergestellt werden. Aufgrund ihrer erhöhten Steifigkeit verringern die DNA-Stränge die Hydrogelquellung und bewahren die Liganden an einer Polymerkette davor, sich mehr als einmal mit dem gleichen tetrameren Protein zu verbinden, was zu einer in ihrem Ausmaß überraschenden wesentlichen Verringerung der Erosion und zu einer Erhöhung der Hydrogel-Lebensdauer führt. Die große Persistenzlänge der doppelsträngigen DNA führt zu einer längeren mittleren Kettenlänge von einem Ende zum anderen mit einer im Vergleich mit PEG zunehmenden Konturlänge, was zu der Möglichkeit der Herstellung von Hydrogelen mit einer reduzierten Menge an Avidin führt. Ein weiterer Vorteil ist die Steuerung der Gelierzeit über die Wärme. Die Stammlösungen können über die Schmelztemperatur der doppelsträngigen DNA erhitzt und dann langsam abgekühlt werden, um die Gelbildung zu verlangsamen.

Die DNA-Hydrogele wurden hergestellt, indem im ersten Schritt komplementäre DNA-Einzelstränge synthetisiert wurden. Jeder Strang wird dann am 5'-Ende biotinyliert. Das Avidin-Protein in dem Gel wird in zwei gleiche Populationen geteilt und dann kurz mit einem DNA-Strang inkubiert. Die Lösungen werden dann über der Schmelztemperatur der DNA erhitzt, gemischt und abgekühlt, um das Hydrogel zu erzeugen. Das Gel kann, wie zuvor beschrieben, als Hydrogelmasse oder als ein dünner Film hergestellt werden. Der dünne Film wird hergestellt, indem zunächst eine kleine Menge einer vorgeheizten Avidin-DNA-Lösung auf die erhitzte Oberfläche pipettiert wird. Eine gleiche Menge der zweiten vorgeheizten Avidin-DNA-Lösung mit der komplementären DNA-Sequenz wird dann auf die Oberfläche pipettiert, gemischt und mit der Pipette über die Oberfläche verteilt, um eine homogene Lösung zu erstellen. Es ist möglich, Filme mit Volumina von Lösungen unter einem Mikroliter herzustellen, so dass eine Massenproduktion von Filmen kostengünstig ist. Die Filme sind auch elastisch und überstehen multiple Zyklen der Dehydratisierung und Rehydratisierung.

Wie die Avidin/Streptavidin/Traptavidin-Biotin-PEG-Hydrogele kann das Gel jede biotinylierte Einheit integrieren. Diese Technologie ermöglicht die Herstellung von DNA-Avidin-Hydrogel-Arrays, die eine beliebige Kombination von Peptiden oder anderen Molekülen in einer breiten Palette von durch den Endbenutzer gewünschten Konzentrationen enthalten. Die Hydrogel-Matrix ist auch individuell einstellbar, so dass für die Anwender Matrices unterschiedlicher Konzentration und Steifigkeit auswählbar sind. Mögliche Anwendungen für eine solche Technologie schließen allgemeines Affinitätsscreening, Drug-Target-Screening, Optimierungsassays für die Zellkulturbedingungen und die kontrollierte Freisetzung der angehängten Ladung ein.

### Beispiel IV: Freisetzung von Biomolekülen aus Hydrogel, kein Teil der Erfindung

Die Biotinylierung stellt die am weitesten verbreitete Methode zur Affinitäts-Erfassung und Immobilisierung von interessanten Biomolekülen dar. Kürzlich wurde ein kovalentes Hydrogelsystem in Kombination mit einer 2-Photonenphotochemischen Reaktion entwickelt, welche die räumliche Erfassung von biotinylierten Substanzen in 3D-Matrices erlaubt. Im Vergleich mit dem chemischen Ansatz durch Pfropfen von Streptavidin an einem Polymernetzwerk führt ein physikalisches Hydrogel, das durch eine Streptavidin (und seine Analoga)-Biotin-Wechselwirkung vernetzt ist, zu einer *in* situ-Gelierung, Ligandimmobilisierung und Verkapselung von Zellen sowie zu einem breiten Konzentrationsbereich zur Beladung mit interessanten biotinylierten Substanzen. Weiterhin kann die resultierende Ladedichte von biotinylierten Biomolekülen genau gesteuert und bestimmt werden, da sie mit dem Verhältnis zwischen biotinyliertem Polymer und interessanten biotinylierten Biomolekülen korreliert. Somit kann das sich ergebende System zu einer unbegrenzten Anzahl von Kombinationen von Liganden in einer 3D-Matrix über einen weiten Konzentrationsbereich (bis zu 1 mM) zur Untersuchung der Zell-Ligand-Wechselwirkung in einer 3D-Umgebung führen. Weiterhin kann das Hydrogel auch als ein Freisetzungssystem biotinylierter niedermolekularer Substanzen und Protein-Therapeutika verwendet werden.

Vorliegend wird ein System zum Lösen biotinylierter Derivate des Immunsuppressivums Cyclosporin A (CsA-Biotin) beschrieben. Das CsA-Biotin ist ähnlich aktiv wie seine Ausgangsverbindung CsA, wie sowohl in dem Calcineurin-Inhibition-Assay als auch in dem immunsuppressiven Assay gegen Jurkat-T-Zellen, menschliche periphere mononukleare Blutzellen und T-Zellen der Maus gezeigt wird. Wenn CsA-Biotin in den Avidin-Biotin-PEG-Hydrogelen eingelagert wird, wird die Freisetzung des immunsuppressiven Wirkstoffs nicht durch die Erosion des Hydrogels, sondern durch die Dissoziation von CsA aus Avidin-Biotin gesteuert. Die mit CsA-azo-Biotin geladenen Hydrogele wurden entweder mit humanem Serum allein oder mit menschlichem Serum mit Biotin bei 37 °C inkubiert. Nachdem die Hydrogele abschließend erodiert waren, wurden die resultierenden Lösungen genutzt, um ein immunsuppressives Assay gegen Maus-T-Zellen durchzuführen.

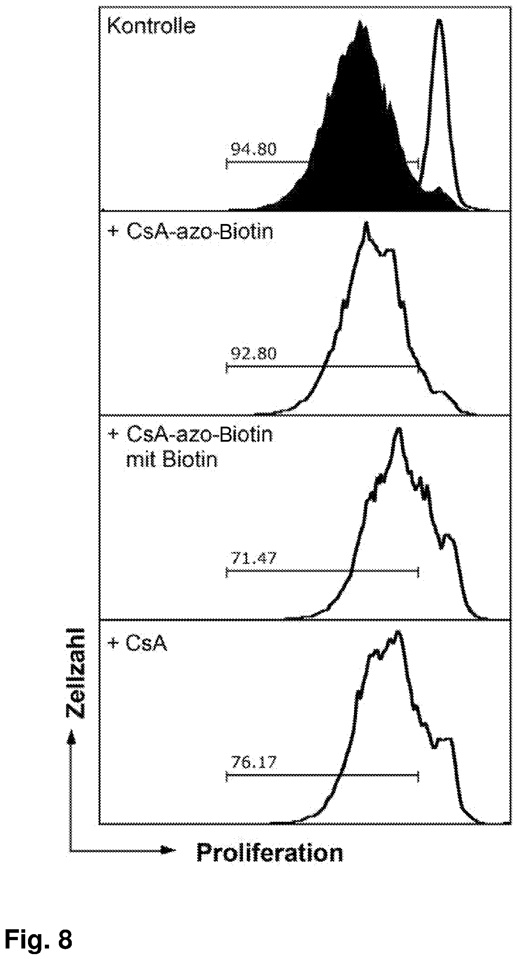

Die Fig. 8 zeigt ein Proliferations-Assay von mittels anit-CD3/CD28-Antikörpern stimulierten Maus T-Zellen unter Verwendung von Durchflusszytometrie in Abhängigkeit von CsA-azo-Biotin, abgegeben vom Hydrogel. Die Kontrolle zeigt die Zellproliferation ohne CsA-Einwirkung. Die weiße Fläche zeigt stimulierte T-Zellen, die schwarze Fläche nicht stimulierte T-Zellen. Je weiter links die Fläche im Diagramm liegt, desto mehr Proliferation findet statt. Eine Menge von 1 µM des immunsuppressiven Medikaments CsA inhibiert die T-Zell-Proliferation. Die T-Zellen wurden zusätzlich mit 1 µM CsA-azo-Biotin, das heißt biotinyliertem CsA, behandelt, welches vom Hydrogel abgegeben wurde. Dieses Hydrogel hatte einen Feststoffgehalt von 9 Massen-%, wobei im Hydrogel ein molares Verhältnis zwischen Avidin und 5kDa PEG-Biotin von 1:2 bestand. Es ist kaum ein Unterschied zur Kontrolle erkennbar. Unter Zuhilfenahme von Biotin kann die Bindung von CsA-azo-Biotin zum Streptavindin/Avidin/Mutante gelöst werden (1 µM CsA-azo-Biotin mit Biotin). Dies führt zu einer Inhibierung der T-Zellen ähnlich der Positivkontrolle CsA. Die Proliferation wurde nach drei Tagen bestimmt.

Wie in der **Fig. 8** gezeigt, ist das erodierte Hydrogel in Gegenwart von Biotin genauso aktiv wie die Ausgangsverbindung CsA bei der Immunsuppression, während das erodierte Hydrogel in Abwesenheit von Biotin keine immunsuppressive Wirkung hat. Interessanterweise haben CsA und das biotinylierte CsA-Analoge eine ähnliche Selektivität zwischen den verschiedenen Subtypen von T-Zellen gezeigt. Sie zeigten deutliche Auswirkungen auf die naiven T-Zellen, während sie keine suppressive Wirkung auf regulatorische T-Zellen haben.

Ohne Zugabe von Biotin können die kleinen Partikel des erodierten Hydrogels noch CsA-Biotin erfassen und verhindern, dass es stark in die Zellen eintritt. Im Gegensatz dazu kann in der Gegenwart von Biotin CsA-Biotin aus Streptavidin dissoziiert werden und eine immunsuppressive Wirkung in den Zellen verursachen. Da das CsA-Biotin, das in dem erodierten Hydrogel erfasst wird, die Peptidyl-Prolyl-cis/trans-Isomerase-Aktivität von Cyclophilin inhibiert, was das primäre Ziel von CsA ist, kann das vorliegende Verfahren zur kontrollierten Wirkstofffunktion führen, entweder durch Hemmung der biologischen Wirkung von extrazellulären Cyclophilinen oder durch die Hemmung des intrazellulären Calcineurins. Darüber hinaus kann durch Austausch von Biotin mit Desthiobiotin oder Iminobiotin sowohl die Gel-Erosion als auch die Freigabe von aktiven Liganden beschleunigt werden.

## Patentansprüche

1. Physikalisches selbstorganisierendes Hydrogelsystem für biotechnologische Anwendungen aus einem nicht-kovalenten Netzwerk auf der Grundlage einer Protein-Ligand-Wechselwirkung, umfassend ein tetrameres Protein als Protein sowie Biotin oder eines seiner Derivate als Ligand, wobei Biotin oder eines seiner Derivate kovalent jeweils an ein Ende einer Polymerkette eines Einzel- oder Doppelstrang-Oligonukleotids konjugiert ist und wobei der Feststoffgehalt bezogen auf das gesamte Hydrogel mindestens 3 Massen-% beträgt und die Konjugate durch das tetramere Protein vernetzt sind, wobei im Hydrogelsystem ein Mischungsverhältnis als molares Äquivalentverhältnis zwischen dem Protein und der Anzahl der terminalen biotinylierten Enden der Polymerketten im Biotin-Polymer-Konjugat von 1:2 bis 1:8 vorliegt.

2. Physikalisches selbstorganisierendes Hydrogelsystem nach Anspruch 1, wobei der Feststoffgehalt, bezogen auf das gesamte Hydrogel mindestens 4 Massen-% beträgt.

3. Physikalisches selbstorganisierendes Hydrogelsystem nach Anspruch 2, wobei der Feststoffgehalt, bezogen auf das gesamte Hydrogel mindestens mindestens 6 Massen-% beträgt.

4. Physikalisches selbstorganisierendes Hydrogelsystem nach Anspruch 3, wobei der Feststoffgehalt, bezogen auf das gesamte Hydrogel mindestens 9 Massen-% beträgt.

5. Physikalisches nicht-kovalentes selbstorganisierendes Hydrogelsystem nach einem der Ansprüche 1 bis 4, wobei als die Konjugate vernetzendes tetrameres Protein Avidin, Streptavidin oder einer Mutante dieser Proteine vorgesehen ist.

6. Physikalisches nicht-kovalentes selbstorganisierendes Hydrogelsystem nach Anspruch 5, **dadurch gekennzeichnet, dass** als die Konjugate vernetzendes tetrameres Protein die Streptavidin-Mutante Traptavidin vorgesehen ist.

7. Physikalisches nicht-kovalentes selbstorganisierendes Hydrogelsystem nach einem der Ansprüche 1 bis 6, **dadurch gekennzeichnet, dass** als Biotinderivate Iminobiotin oder Desthiobiotin vorgesehen sind.

8. Physikalisches nicht-kovalentes selbstorganisierendes Hydrogelsystem nach einem der Ansprüche 1 bis 7, **dadurch gekennzeichnet, dass** als Oligonukleotid für die Polymerketten ein verzweigtes Oligonukleotid, das heißt ein Oligonukleotid mit mehr als zwei Enden eingesetzt wird.

9. Physikalisches nicht-kovalentes selbstorganisierendes Hydrogelsystem nach einem der Ansprüche 1 bis 7, **dadurch gekennzeichnet, dass** die Polymerketten jeweils aus einer doppelsträngigen DNA bestehen.

10. Physikalisches nicht-kovalentes selbstorganisierendes Hydrogelsystem nach einem der Ansprüche 1 bis 9, **dadurch gekennzeichnet, dass** dieses durch Einfügen von Peptidsequenzen in die Polymerketten oder durch Zugabe von biotinylierten Peptiden, biotinylierten Wirkstoffen, biotinylierten Oligosacchariden, biotinylierten Oligonukleotiden oder biotinylierten Proteinen in das Hydrogel modifiziert ist.

11. Verfahren zur Herstellung eines Hydrogels nach einem der Ansprüche 1 bis 10, bei dem die zu vernetzenden Komponenten, das heißt das Protein und das Konjugat, bei einem Feststoffgehalt von mindestens 3 Massen-% und in einem molaren Äquivalentverhältnis zwischen dem Protein und der Anzahl der terminalen biotinylierten Enden der Polymerketten im Biotin-Polymer-Konjugat von 1:2 bis 1:8 miteinander gemischt werden.

12. Verwendung eines physikalischen nicht-kovalenten selbstorganisierenden Hydrogelsystems nach einem der Ansprüche 1 bis 10 zur Kultivierung von Zellen.

13. Verwendung eines physikalischen nicht-kovalenten selbstorganisierenden Hydrogelsystems nach einem der Ansprüche 1 bis 10 zur Einkapselung von Zellen.

## Claims

1. A physical self-organizing hydrogel system for biotechnological applications composed of a non-covalent network on the basis of a protein-ligand interaction includes a tetrameric protein as protein and biotin or a derivative thereof as a ligand,
wherein biotin or derivative is covalently conjugated to an end of a polymer chain of a single-or double-strand oligonucleotide and wherein the solid content in relation to the entire hydrogel is at least 3% and the conjugates are crosslinked by the tetrameric protein, wherein a mixture ratio in the hydrogel system comprising a molar equivalent ratio between the protein and the number of terminal biotinylated ends of the polymer chains in the biotin-polymer conjugate of 1:2 to 1:8.

2. A physical self-organizing hydrogel system according to claim 1
wherein the solid content in relation to the entire hydrogel is at least 4% of the mass.

3. A physical self-organizing hydrogel system according to claim 2
wherein the solid content in relation to the entire hydrogel is at least 6% of the mass.

4. A physical self-organizing hydrogel system according to claim 3,
wherein the solid content in relation to the entire hydrogel is at least 9% of the mass.

5. A physical non-covalent self-organizing hydrogel system according to one of claims 1 to 4, wherein Avidin, Streptavidin or a mutant of these proteins is intended as the tetrameric protein to cross-link the conjugates.

6. A physical non-covalent self-organizing hydrogel system according to claim 5, **characterised in that** the Streptavidin-mutant Traptavidin is intended as the tetrameric protein to cross-link the conjugates.

7. A physical non-covalent self-organizing hydrogel system according to one of claims 1 to 6, **characterised in that** Iminobiotin or Desthiobiotin are intended as the biotin derivatives.

8. A physical non-covalent self-organizing hydrogel system according to one of claims 1 to 7, **characterised in that** a branched oligonucleotide, i.e. an oligonucleotide with more than two terminal ends, is intended as the oligonucleotide for the polymer chains.

9. A physical non-covalent self-organizing hydrogel system according to one of claims 1 to 7, **characterised in that** the polymer chains each comprise double-stranded DNA.

10. A physical non-covalent self-organizing hydrogel system according to one of claims 1 to 9, **characterised in that** it is modified by means of inserting peptide sequences into the polymer chain or by the addition of biotinylated peptides, biotinylated active ingredients, biotinylated oligosaccharides, biotinylated oligonucleotides or biotinylated proteins into the hydrogel.

11. A method for producing a hydrogel according to one of claims 1 to 10 wherein the components to be cross-linked, i.e. the protein and the conjugate, are mixed together wherein the solids content of the hydrogel is at least 3% and the molar equivalent ratio between the protein and the number of biotinylated terminal ends of the polymer chains in the biotin-polymer conjugate is 1:2 to 1:8.

12. A method of using a physical non-covalent self-organizing hydrogel system according to one of claims 1 to 10 for cultivating cells.

13. A method of using a physical non-covalent self-organizing hydrogel system according to one of claims 1 to 10 for encapsulating cells.

## Revendications

1. Système d'hydrogel auto-assemblant physique pour applications biotechnologiques, issu d'un réseau non covalent basé sur une interaction entre la protéine et le ligand, comprenant une protéine tétramère en tant que protéine, ainsi qu'une biotine ou l'un de ses dérivés en tant que ligand, la biotine ou l'un de ses dérivés covalent étant conjugué à une extrémité d'une chaîne polymère d'un oligonucléotide simple brin ou double brin et la teneur en matières solides par rapport à l'hydrogel représentant au moins 3 % en masse et les conjugués étant liés par la protéine tétramère,
le système d'hydrogel présentant un rapport de mélange en tant que rapport d'équivalent molaire entre la protéine et le nombre d'extrémités biotinylées terminales des chaînes polymères dans le conjugué biotine-polymère de 1:2 à 1:8.

2. Système d'hydrogel auto-assemblant physique selon la revendication 1 tel que la teneur en matières solides par rapport à l'hydrogel total représente au moins 4 % en masse.

3. Système d'hydrogel auto-assemblant physique selon la revendication 2 tel que la teneur en matières solides par rapport à l'hydrogel total représente au moins 6 % en masse.

4. Système d'hydrogel auto-assemblant physique selon la revendication 3 tel que la teneur en matières solides par rapport à l'hydrogel total représente au moins 9 % en masse.

5. Système d'hydrogel auto-assemblant non covalent physique selon l'une des revendications 1 à 4 tel que les conjugués envisagés de la protéine tétramère liée sont l'avidine, la streptavidine ou une forme mutante de ces protéines.

6. Système d'hydrogel auto-assemblant non covalent physique selon la revendication 5, **caractérisé en ce que** la forme mutante de la streptavidine, la traptavidine, est envisagée en tant que conjugué de la protéine tétramère liée.

7. Système d'hydrogel auto-assemblant non covalent physique selon l'une des revendications 1 à 6, **caractérisé en ce que** l'iminobiotine ou la desthiobiotine sont envisagées en tant que dérivé de la biotine.

8. Système d'hydrogel auto-assemblant non covalent physique selon l'une des revendications 1 à 7, **caractérisé en ce que** un oligonucléotide ramifié est utilisé en tant qu'oligonucléotide pour les chaînes polymères, c'est-à-dire un oligonucléotide avec plus de deux extrémités.

9. Système d'hydrogel auto-assemblant non covalent physique selon l'une des revendications 1 à 7, **caractérisé en ce que** les chaînes polymères se composent d'un ADN double brin.

10. Système d'hydrogel auto-assemblant non covalent physique selon l'une des revendications 1 à 9, **caractérisé en ce que** ce dernier est modifié par ajout de séquences peptidiques dans les chaînes polymères ou par ajout de peptides biotinylés, de matières biotinylées, d'oligosaccharides biotinylés, d'oligonucléotides biotinylés ou de protéines biotinylées dans l'hydrogel.

11. Procédé de fabrication d'un hydrogel selon l'une des revendications 1 à 10 selon lequel les composants à lier, c'est-à-dire la protéine et le conjugué, sont mélangés pour une teneur en matières solides d'au moins 3 % en masse et dans un rapport d'équivalent molaire entre la protéine et le nombre d'extrémités biotinylées terminales des chaînes polymères du conjugué biotine-polymère de 1:2 à 1:8.

12. Utilisation d'un système d'hydrogel auto-assemblant non covalent physique selon l'une des revendications 1 à 10 pour la culture de cellules.

13. Utilisation d'un système d'hydrogel auto-assemblant non covalent physique selon l'une des revendications 1 à 10 pour l'encapsulation de cellules.
